# EUROPEAN PATENT SPECIFICATION

(11) **EP 1 802 336 B1**
(45) Date of publication and mention of the grant of the patent: **07.09.2011**
(21) Application number: 05808091.2
(22) Date of filing: 13.10.2005
(51) Int. Cl.: A61K 39/015

(54) **MALARIA PRIME/BOOST VACCINES**
PRIME-BOOST-IMPFSTOFFE GEGEN MALARIA
VACCIN INITIAL ET DE RAPPEL CONTRE LA MALARIA

(30) Priority: 14.10.2004 US 619056 P; 14.10.2004 EP 04105035
(43) Date of publication of application: 04.07.2007
(73) Proprietor: Crucell Holland B.V., 2333 CN Leiden (NL); GlaxoSmithKline Biologicals SA, 1330 Rixensart (BE); The Government of the United States, as represen- ted by the Secretary of the Army, on behalf of the Walter Reed Army Institute of Research, Silver Spring, MD 20910-7500 (US)
(72) Inventor: PAU, Maria Grazia, NL-2333 CW Leiden (NL); GOUDSMIT, Jaap, NL-1075 CX Amsterdam (NL); COHEN, Joseph D., GlaxoSmithKline, B-1330 Rixensart (BE); DUBOIS, Patrice M., GlaxoSmithKline, B-1330 Rixensart (BE); STEWART, V. Ann, Silver Spring, Maryland 20910-7500 (US); HEPPNER, Donald, Silver Spring, Maryland 20910-7500 (US)
(74) Representative: Stephen, Robert John
(86) International application number: PCT/EP2005/055209
(87) International publication number: WO 2006/040334

(56) References cited:
- WO-A-93/10152
- WO-A-2004/055187
- STOUTE J A ET AL: "Long-term efficacy and immune responses following immunization with the RTS,S malaria vaccine." THE JOURNAL OF INFECTIOUS DISEASES. OCT 1998, vol. 178, no. 4, October 1998 (1998-10), pages 1139-1144, XP008046110 ISSN: 0022-1899
- GARÇON NATHALIE ET AL: "Development of RTS,S/AS02: a purified subunit-based malaria vaccine candidate formulated with a novel adjuvant." EXPERT REVIEW OF VACCINES. APR 2003, vol. 2, no. 2, April 2003 (2003-04), pages 231-238, XP008046132 ISSN: 1476-0584
- SUN PEIFANG ET AL: "Protective immunity induced with malaria vaccine, RTS,S, is linked to Plasmodium falciparum circumsporozoite protein-specific CD4+ and CD8+ T cells producing IFN-gamma." JOURNAL OF IMMUNOLOGY, vol. 171, no. 12, 15 December 2003 (2003-12-15), pages 6961-6967, XP002325853 ISSN: 0022-1767
- STOUTE J A ET AL: "A PRELIMINARY EVALUATION OF A RECOMBINANT CIRCUMSPOROZOITE PROTEIN VACCINE AGAINST PLASMODIUM FALCIPARUM MALARIA" NEW ENGLAND JOURNAL OF MEDICINE, THE, MASSACHUSETTS MEDICAL SOCIETY, WALTHAM, MA, US, vol. 336, no. 2, 9 January 1997 (1997-01-09), pages 86-91, XP000990284 ISSN: 0028-4793
- DOHERTY J F ET AL: "A Phase I safety and immunogenicity trial with the candidate malaria vaccine RTS,S/SBAS2 in semi-immune adults in The Gambia" AMERICAN JOURNAL OF TROPICAL MEDICINE AND HYGIENE, vol. 61, no. 6, December 1999 (1999-12), pages 865-868, XP002325854 ISSN: 0002-9637
- KESTER KENT E ET AL: "Efficacy of recombinant circumsporozoite protein vaccine regimens against experimental Plasmodium falciparum malaria" JOURNAL OF INFECTIOUS DISEASES, vol. 183, no. 4, 15 February 2001 (2001-02-15), pages 640-647, XP002325855 ISSN: 0022-1899
- SCHWENK ROBERT ET AL: "Opsonization by antigen-specific antibodies as a mechanism of protective immunity induced by Plasmodium falciparum circumsporozoite protein-based vaccine." PARASITE IMMUNOLOGY (OXFORD), vol. 25, no. 1, January 2003 (2003-01), pages 17-25, XP002325856 ISSN: 0141-9838
- BRUNA-ROMERO O ET AL: "Enhanced protective immunity against malaria by vaccination with a recombinant adenovirus encoding the circumsporozoite protein of Plasmodium lacking the GPI-anchoring motif" VACCINE, BUTTERWORTH SCIENTIFIC. GUILDFORD, GB, vol. 22, no. 27-28, 9 September 2004 (2004-09-09), pages 3575-3584, XP004526937 ISSN: 0264-410X
- WALSH DOUGLAS S ET AL: "Safety and immunogenicity of RTS,S+TRAP malaria vaccine, formulated in the AS02A adjuvant system, in infant rhesus monkeys" AMERICAN JOURNAL OF TROPICAL MEDICINE AND HYGIENE, vol. 70, no. 5, May 2004 (2004-05), pages 499-509, XP008046107 ISSN: 0002-9637
- HEPPNER D G ET AL: "Towards an RTS,S-based, multi-stage, multi-antigen vaccine against falciparum malaria: progress at the Walter Reed Army Institute of Research" VACCINE, BUTTERWORTH SCIENTIFIC. GUILDFORD, GB, vol. 23, no. 17-18, 18 March 2005 (2005-03-18), pages 2243-2250, XP004777532 ISSN: 0264-410X

## Description

### FIELD OF THE INVENTION

The invention relates to the field of medicine. Specifically, the invention relates to novel prime/boost vaccine strategies using recombinantly produced adenoviral vectors and purified proteins in the context of an adjuvant for the prevention of falciparum malaria.

### BACKGROUND OF THE INVENTION

Malaria currently represents one of the most prevalent infections in tropical and subtropical areas throughout the world. Per year, malaria infections kill up to 2.7 million people in developing and emerging countries. The widespread occurrence and elevated incidence of malaria are a consequence of the increasing numbers of drug-resistant parasites and insecticide-resistant parasite vectors. Other factors include environmental and climatic changes, civil disturbances and increased mobility of populations.

Malaria is caused by mosquito-borne hematoprotozoan parasites belonging to the genus *Plasmodium.* Four species of *Plasmodium* protozoa *(P. falciparum, P. vivax, P. ovale* and *P.malariae)* are responsible for the disease in man; many others cause disease in animals, such as *P.yoelii* and *P.berghei. P.falciparum* accounts for the majority of infections in humans and is the most lethal type. Malaria parasites have a life cycle consisting of four separate stages. Each one of these stages is able to induce specific immune responses directed against the parasite and the correspondingly occurring stage-specific antigens, yet naturally induced malaria does not protect against reinfection.

Malaria parasites are transmitted to man by several species of female *Anopheles* mosquitoes. Infected mosquitoes inject the sporozoite form of the malaria parasite into the mammalian bloodstream. Sporozoites remain for few minutes in the circulation before invading hepatocytes. At this stage the parasite is located in the extra-cellular environment and is exposed to antibody attack, mainly directed to the circumsporozoite (CS) protein, a major component of the sporozoite surface. Once in the liver, the parasite replicates and develops into a schizont. During this stage, the invading parasite will undergo asexual multiplication, producing up to 20,000 daughter merozoites per infected cell. During this intra-cellular stage of the parasite, main players of the host immune response are T-lymphocytes, especially CD8+ T-lymphocytes (Romero et al. 1989). After about one week of liver infection, thousands of merozoites are released into the bloodstream and enter red blood cells (RBC's), becoming targets of antibody-mediated immune response and T-cell secreted cytokines. After invading the erythrocytes, the merozoites undergo several stages of replication, transforming into trophozoites, and schizonts, which rupture to produce a new generation of merozoites that subsequently infect new RBC's. The erythrocytic stage is associated with overt clinical disease. A smaller number of trophozoites may develop into male or female gametocytes, which are the parasite's sexual stage. When susceptible mosquitoes ingest gametocytes, the fertilization of these gametes leads to zygote formation and subsequent transformation into ookinetes, then into oocysts, and finally into sporozoites, which migrate to the salivary gland to complete the cycle.

The two major arms of the pathogen-specific immune response that occur upon entry of the parasite into the body are cellular and humoral. The one arm, the cellular response, relates to CD8+ and CD4+ T cells that participate in the immune response. Cytotoxic T lymphocytes (CTL's) express CD8 and are able to specifically kill infected cells that express pathogenic antigens on their surface. CD4+ T cells or T helper cells support the development of CTL's, produce various cytokines, and also help induce B cells to divide and produce antibodies specific for the antigens. During the humoral response, B cells specific for a particular antigen become activated, replicate, differentiate and produce antigen-specific antibodies.

Both arms of the immune response are relevant for protection against a malarial infection. When infectious sporozoites travel to the liver and enter the hepatocytes, the sporozoites become intracellular pathogens, spending little time outside the infected cells. At this stage, CD8+ T cells and CD4+ T cells are especially important because these T cells and their cytokine products, such as interferon-γ (IFN-γ), contribute to the killing of infected host hepatocytes. Elimination of the intracellular liver parasites in the murine malaria model is found to be dependent upon CD8+ T cell responses directed against peptides expressed by liver stage parasites (Hoffman and Doolan, 2000). Depletion of CD8+ T cells abrogates protection against sporozoite challenge, and adoptive transfer of CD8+ T cells to naive animals confers protection.

When a malarial infection reaches the erythrocytic stage in which merozoites replicate in RBC's, the merozoites are also found circulating freely in the bloodstream. Because the erythrocyte does not express either Class I or II MHC molecules required for cognate interaction with T cells, it is thought that antibody responses are most relevant at this stage. In conclusion, a possible malaria vaccine approach would be most beneficial if it would induce a strong cellular immune response as well as a strong humoral immune response to tackle the different stages in which the parasite occurs in the human body.

Current approaches to malaria vaccine development can be classified according to the different developmental stages of the parasite, as described above. Three types of possible vaccines can be distinguished:
- Pre-erythrocytic vaccines, which are directed against sporozoites and/or schizont-infected hepatocytes. Historically, this approach has been dominated by (CS)-based strategies. Since the pre-erythrocytic phase of infection is asymptomatic, a pre-erythrocytic vaccine should ideally confer sterile immunity, mediated by humoral and cellular immune response, and completely prevent latent malaria infection.
- Asexual blood stage vaccines, which are directed against either the infected RBC or the merozoite itself, are designed to minimize clinical severity. These vaccines should reduce morbidity and mortality and are meant to prevent the parasite from entering and/or developing in the erythrocytes.
- Transmission-blocking vaccines, which are designed to hamper the parasite development in the mosquito host. This type of vaccine should favor the reduction of population-wide malaria infection rates.

Finally, the feasibility of developing combination malaria vaccines that target multiple stages of the parasite life cycle is being pursued in so-called multicomponent and/or multi-stage vaccines.

Currently no commercially available vaccine against malaria is available, although the development of vaccines against malaria was initiated more than 30 years ago. Immunization of rodents, non-human primates and humans with radiation-attenuated sporozoites conferred protection against a subsequent challenge with viable sporozoites (Nussenzweig et al. 1967; Clyde et al. 1973). However, so far the expense and the lack of a feasible large-scale culture system for the production of irradiated sporozoites has prevented the widespread application of such vaccines (Luke et al. 2003).

To date the most promising vaccine candidates tested in humans have been based on a small number of sporozoite surface antigens. The CS protein is the only *P.falciparum* antigen demonstrated to consistently prevent malaria when used as the basis of active immunization in humans against mosquito-borne infection, albeit at levels that are often insufficient. Theoretical analysis has indicated that the vaccine coverage as well as the vaccine efficiency should be above 85%, or otherwise mutants that are more virulent may escape in endemic areas (Gandon et al. 2001).

One way of inducing an immune response in a mammal is by administering an infectious vector, which harbors a nucleic acid encoding the antigen in its genome. One such carrier is a recombinant adenovirus, which has been rendered replication-defective by removal of regions within the genome that are normally essential for replication, such as the E1 region. Examples of recombinant adenoviruses that comprise genes encoding antigens are known in the art (WO 96/39178). For instance, HIV-derived antigenic components have been demonstrated to yield an immune response if delivered by recombinant adenoviruses (WO 01/02607; WO 02/22080; US 6,733,993). In malaria, recombinant adenovirus-based vaccines have been developed. These vectors express the entire CS protein of *P.yoelii,* one of the mouse malaria models, and these vectors have been shown to be capable of inducing sterile immunity in mice in response to a single immunizing dose (Bruña-Romero et al. 2001). It has been demonstrated that CD8+ T cells primarily mediate this adenovirus-induced protection.

Since a high percentage of individuals have pre-existing immunity against the generally used adenoviral vectors such as adenovirus serotype 5 (Ad5), new technologies were developed in the art, wherein recombinant replication-defective adenoviruses were based on serotypes that encountered pre-existing immunity in the form of neutralizing antibodies only in a small percentage of healthy individuals. These serotypes are generally referred to as low-neutralized serotypes, or rare serotypes. It was found that Ad11, Ad24, Ad26, Ad34, Ad35, Ad48, Ad49 and Ad50 were particularly useful (WO 00/70071; WO 02/40665; WO 2004/037294; WO 2004/083418; Vogels et al. 2003).

A DNA-based vaccine containing a plasmid that expresses the *P.falciparum* CS protein was developed by Vical, Inc. San Diego, CA, USA and the Naval Medical Research Center (Horn et al. 1995). Studies in a mouse model demonstrated induction of antigen-specific CTL and antibody responses following immunization with plasmid DNA (Doolan et al. 1998). However, thus far the sole use of DNA vaccines have proved suboptimal for induction of protective immune responses in humans. Using the DNA vaccine it was found that vaccinated volunteers did not develop antibodies against the CS protein as assessed by indirect fluorescent antibody test (IFAT) against airdried sporozoites and ELISA against recombinant and synthetic peptides (Wang et al. 2001), although their CTL responses were significant.

In contrast, the RTS,S (purified protein) malaria vaccine approach (Gordon et al. 1995; US 6,306,625; WO 93/10152) is able to induce a robust antibody response to the CS protein (Kester et al. 2001; Stoute et al. 1997 and 1998), while it is also a potent inducer of Th1 type cellular and humoral immunity. Most importantly, this vaccine repeatably protects approximately half of the recipients. However, the protection elicited by RTS,S is of short duration (Stoute et al. 1998). Immunization with RTS,S induces anti-CS antibodies and CD4+ T cell-dependent IFN-γ responses, but poor CD8+ T cell-dependent CTL or IFN-γ responses (Lalvani et al. 1999). However, these minimal CD8+ responses that are produced have been demonstrated to correlate with protection in human trials (Sun et al. 2003). Thus, a rational improvement would focus on enhancement of the induction of CD8+ T cell responses to CS induced by RTS,S.

Stoute et al. The Journal of Infectious Disease, 1998, vol. 178, pages 1139-44 and Garçon et al. Expert Review of Vaccines, April 2003, vol. 2, no. 2, pages 231-238 disclose efficacy of the malaria sporozoite vaccine candidate RTS,S formulated with an oil-in-water emulsion plus the immunostimulants monophosphoryl lipid A and the saponin derivative QS2.

WO 2004/055187 discloses replication-defective recombinant adenovirus comprising a heterologous nucleic acid encoding the CS antigen of *Plasmodium* for use as a vaccine.

The challenge of developing a falciparum malaria vaccine that has a protective efficacy of at least 85% has not yet been met. The task is particularly difficult because, unlike with other often fatal diseases such as measles or smallpox, prior malaria exposure and the development of natural immunity is not protective against subsequent malaria infection. Of all vaccine candidates and vaccine delivery strategies tested to date, only RTS,S has consistently provided some level of protection. Other tested candidates have either been inadequately immunogenic, or immunogenic but inadequately protective. This application describes a strategized combination of vaccine formulations designed to take advantage of the optimal serologic immunogenicity of the protein/adjuvant approach together with an excellent induction of cellular responses provided by recombinant replication-defective adenoviral vectors.

### BRIEF DESCRIPTION OF THE DRAWINGS

Figure 1. Heterologous prime/boost vaccination regimens, followed by measuring the T cell response in IFN-γ ELISPOT analyses related to the C-terminus of CS. Response was measured two weeks after final boost. Horizontal bars represent geometric means.
Figure 2. T cell response measured in IFN-γ ELISPOT analyses related to the C-terminus of CS. Response was measures three months after final boost. Horizontal bars represent geometric means.

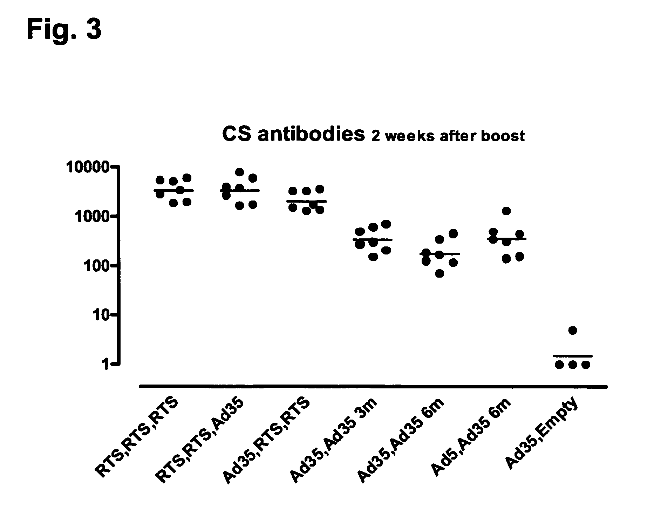

Figure 3. Antibody response measured in ELISA, related to the repeat region of CS, two weeks after boost. Horizontal bars represent geometric means.

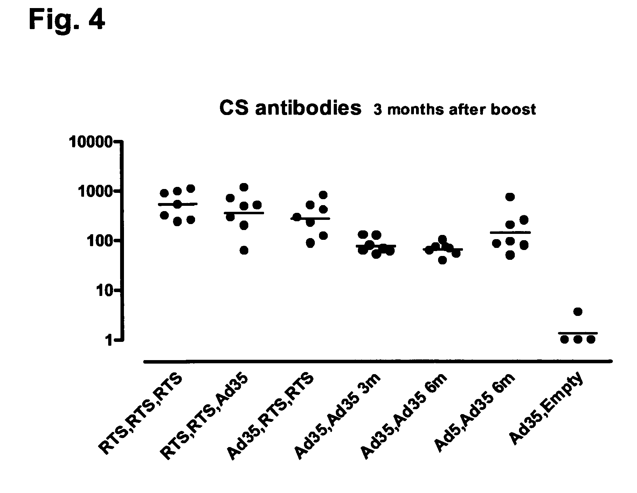

Figure 4. Antibody response measured in ELISA, related to the repeat region of CS, three months after boost. Horizontal bars represent geometric means.
Figure 5. T cell response, in experiments priming with a recombinant Ad35-CS vector and boosting with RTS,S or Ad35-CS, measured by IFN-γ ELISpot after two weeks (left) or after three months (right). The homologous prime/boost/boost regimen RTS,S/RTS,S/RTS,S was used as a reference.
Figure 6. Antibody response, in experiments priming with a recombinant Ad35-CS vector and boosting with RTS,S or Ad35-CS, measured by ELISA after two weeks (left) or after three months (right). The homologous prime/boost/boost regimen RTS,S/RTS,S/RTS,S was used as a reference.
Figure 7. T cell response, in experiments boosting with a recombinant Ad35-CS vector and priming with RTS,S or Ad5-CS, measured after two weeks (left) or after three months (right). The homologous prime/boost/boost regimen RTS,S/RTS,S/RTS,S was used as a reference.
Figure 8. Antibody response, in experiments boosting with a recombinant Ad35-CS vector and priming with RTS,S or Ad5-CS, measured after two weeks (left) or after three months (right). The homologous prime/boost/boost regimen RTS,S/RTS,S/RTS,S was used as a reference.
Figure 9. T cell response measured in IFN-γ ELISPOT analyses related to the N-terminus of CS, two weeks after boost. Horizontal bars represent geometric means.
Figure 10. T cell response measured in IFN-γ ELISPOT analyses related to the N-terminus of CS, three months after boost. Horizontal bars represent geometric means.
Figure 11. T cell response to the N-terminus, in experiments priming with a recombinant Ad35-CS vector and boosting with RTS,S or Ad35-CS, measured after two weeks (left) or after three months (right). The homologous prime/boost/boost regimen RTS,S/RTS,S/RTS,S was used as a reference.
Figure 12. T cell response to the N-terminus, in experiments boosting with a recombinant Ad35-CS vector and priming with RTS,S or Ad5-CS, measured after two weeks (left) or after three months (right). The homologous prime/boost/boost regimen RTS,S/RTS,S/RTS,S was used as a reference.

### SUMMARY OF THE INVENTION

The invention relates to a kit of parts comprising a replication-defective recombinant adenovirus in a suitable excipient, said adenovirus comprising a heterologous nucleic acid encoding a circumsporozoite (CS) antigen from a malaria-causing parasite; and an adjuvated proteinaceous antigen, preferably also from a malaria-causing parasite; wherein said recombinant adenovirus is selected from the group consisting of human adenovirus serotype 11, 24, 26, 34, 35, 48, 49 and 50. A preferred proteinaceous antigen comprises RTS,S. The preferred malaria-causing parasite is *Plasmodium falciparum.*

In another embodiment the invention also relates to the use of a replication-defective recombinant adenovirus comprising a heterologous nucleic acid encoding a CS antigen from a malaria-causing parasite, and an adjuvated proteinaceous antigen, preferably from a malaria-causing parasite such as *Plasmodium falciparum,* in the manufacture of a medicament for the treatment or prevention of malaria, wherein said recombinant adenovirus is a simian adenovirus or a human adenovirus serotype 11, 24, 26, 34, 35, 48, 49 or 50.

The invention discloses certain preferred prime-boost regimens, wherein it is preferred that the replication-defective recombinant adenovirus is used as a priming composition and the adjuvated proteinaceous antigen is used as a boosting composition.

The invention also relates to a method of vaccinating a mammal for a malaria infection comprising the steps of priming said mammal with a replication-defective recombinant adenovirus in a suitable excipient, said adenovirus comprising a heterologous nucleic acid encoding a CS antigen from a malaria-causing parasite; and boosting said mammal with an adjuvated proteinaceous antigen, preferably RTS,S.

### DETAILED DESCRIPTION

The present invention relates to a kit of parts comprising a replication-defective recombinant adenovirus in a pharmaceutically acceptable excipient, said adenovirus comprising a heterologous nucleic acid encoding a circumsporozoite (CS) antigen from a malaria-causing parasite; and an adjuvated proteinaceous antigen; wherein said recombinant adenovirus is selected from the group consisting of human adenovirus serotype 11, 24, 26, 34, 35, 48, 49 and 50. Preferably, said recombinant adenovirus is human adenovirus serotype 35. Also preferred is a kit according to the invention wherein said proteinaceous antigen comprises a CS protein, or an immunogenic fragment thereof, from a malaria-causing parasite. Said proteinaceous antigen comprises preferably a hybrid protein of CS protein or an immunogenic fragment thereof fused to the surface antigen from hepatitis B virus (HbsAg), in the form of lipoprotein particles with HbsAg. In a further preferred embodiment, the proteinaceous antigen comprises RTS,S. It is also preferred that said proteinaceous antigen is adjuvated with QS21 and 3D-MPL, preferably in a formulation with cholesterol-containing liposomes.

Although it is known in the art that different parasites cause malaria in humans, one embodiment of the present invention is a kit of parts according to the invention, wherein said malaria-causing parasite is *Plasmodium falciparum.*

For proper immune responses it is preferred that said heterologous nucleic acid is codon-optimized for increased production of the encoded protein in a mammal, preferably a human. The recombinant adenovirus may be present in a mixture with an adjuvant.

The applicability of simian adenoviruses for use in human gene therapy or vaccines is well appreciated by those of ordinary skill in the art. Besides this, other non-human adenoviruses such as canine and bovine adenoviruses were found to infect human cells in vitro and are therefore also applicable for human use since their seroprevalence is low in human samples. Thus, the invention also relates to a kit of parts comprising a replication-defective recombinant simian, canine or bovine adenovirus in a pharmaceutically acceptable excipient, said adenovirus comprising a heterologous nucleic acid encoding a codon-optimized circumsporozoite (CS) antigen from *Plasmodium falciparum;* and an adjuvated proteinaceous antigen comprising RTS,S, wherein it is preferred that said proteinaceous antigen is adjuvated with QS21 and 3D-MPL, preferably in a formulation with cholesterol-containing liposomes.

It is disclosed by the present invention that certain prime-boost regimens provide an unexpected and striking result with respect to immune responses if the different components of the kit of parts disclosed are administered in a certain order. Thus, the invention also relates to a kit of parts according to the invention, wherein said replication-defective recombinant adenovirus is a priming composition and said adjuvated proteinaceous antigen is a boosting composition. The immune response triggered by a single administration (prime) of a vaccine is often not sufficiently potent and/or persistent to provide effective protection. Repeated administration (boost) can significantly enhance humoral and cellular responses to vaccine antigens (e.g., see Estcourt et al. 2002).

The invention also relates to the use of a replication-defective recombinant adenovirus comprising a heterologous nucleic acid encoding a CS antigen from a malaria-causing parasite, and an adjuvated proteinaceous antigen in the manufacture of a medicament for the treatment or prevention of malaria, wherein said recombinant adenovirus is a simian, a canine, a bovine adenovirus, or a human adenovirus serotype 11, 24, 26, 34, 35, 48, 49 or 50, wherein it is preferred that said replication-defective recombinant adenovirus is used as a priming composition and said adjuvated proteinaceous antigen is used as a boosting composition. According to one embodiment of the invention, it relates to a use according to the invention, wherein the proteinaceous antigen comprises a CS protein, or an immunogenic fragment thereof, from a malaria-causing parasite, preferably *Plasmodium falciparum.* Said proteinaceous antigen preferably comprises a hybrid protein of CS protein or an immunogenic fragment thereof fused to the surface antigen from hepatitis B virus (HbsAg), in the form of lipoprotein particles with HbsAg. RTS,S is a preferred adjuvated proteinaceous antigen, while a preferred adjuvant is QS21 and 3D-MPL, preferably in a formulation with cholesterol-containing liposomes.

For optimal expression followed by optimal immune responses in mammals, preferably humans, the heterologous nucleic acid used in the present invention is codon-optimized for increased production of the encoded protein in a mammal, preferably a human.

In yet another embodiment, the present invention relates to a method of vaccinating a mammal for a malaria infection comprising the steps of priming said mammal with a replication-defective recombinant adenovirus in a pharmaceutically acceptable excipient, said adenovirus comprising a heterologous nucleic acid encoding a CS antigen from a malaria-causing parasite; and boosting said mammal with an adjuvated proteinaceous antigen comprising a hybrid protein of CS protein or an immunogenic fragment thereof fused to HbsAg, in the form of lipoprotein particles with HbsAg. The proteinaceous antigen preferably comprises RTS,S, wherein the preferred adjuvant is QS21 and 3D-MPL, preferably in a formulation with cholesterol-containing liposomes, whereas the preferred malaria-causing parasite is *Plasmodium falciparum.*

Preferred adenoviruses that are used to produce recombinant adenovirus and used in the methods of the present invention may be human or non-human adenoviruses such as simian-, canine- and bovine adenoviruses, since it is highly preferred to use adenoviruses that do not encounter pre-existing immunity in the (human) host to which the recombinant virus is to be administered. Simian adenoviruses and certain serotypes of human adenoviruses are highly suited for this, as disclosed herein. Preferred human adenoviruses that are used for the methods, uses and kit-of parts according to the invention are human adenovirus serotypes 11, 24, 26, 34, 35, 48, 49 and 50.

The invention also relates to a method of vaccinating a mammal for a malaria infection using a kit of parts according to the invention. If a kit of parts according to the invention is used for vaccinating a mammal for a malaria infection using a preferred prime-boost regimen as disclosed herein, the boost is preferably followed by one or more subsequent boosts.

The present invention relates to the use of recombinant adenovirus as a carrier of at least one malaria antigen and used in heterologous combination with one adjuvated protein in a prime/boost regimen. It has surprisingly been found that the combination of a viral vector and an adjuvated protein in a heterologous prime/boost regimen provides a superior immune response in primates in terms of initial T cell responses and longevity of the immune responses. In particular, it has been found that priming a mammal with a viral vector carrying a nucleic acid encoding an antigen followed by a subsequent boosting, either by single or multiple injection of adjuvated proteinaceous antigen provides superior results in terms of qualitative and/or quantitative immune responses. Preferred viral vectors are adenoviral vectors, more preferably human adenoviral vectors, and even more preferably human adenoviral vectors that encounter low levels of neutralizing activity in the mammalian host to which it is administered. Highly preferred serotypes are adenovirus 11, 24, 26, 34, 35, 48, 49 and 50.

According to one preferred embodiment, the proteinaceous antigen and the antigen encoded by the viral vector are malaria antigens, more preferably the *Plasmodium falciparum* circumsprorozoite (CS) protein, or immunogenic derivatives and/or fragments thereof. As one example of this concept, the polypeptide encoded by the viral vector comprises the nucleic acid encoding the P. *falciparum* CS protein, including the N-terminal part, the central part repeat region, and the C-terminal part (with a deletion of the 14 most C-terminal amino acids: the GPI anchor sequence), while the proteinaceous antigen comprises the construct RTS,S, which lacks the N-terminal region.

The adjuvated proteinaceous antigen for use in any or all aspects of the invention may comprise the CS protein from *P. falciparum,* or an immunogenic fragment thereof, which may be in the form of a fusion protein. For example, the antigen may comprise a hybrid protein of CS protein or an immunogenic fragment fused to the surface antigen from hepatitis B virus (HBsAg), which hybrid protein may be expressed in prokaryotic or eukaryotic host cells and may take the form of lipoprotein particles. The fusion protein may comprise for example substantially all the C-terminal portion of the CS protein, four or more tandem repeats of the immunodominant region, and the surface antigen from hepatitis B virus (HBsAg). For example the hybrid protein comprises a sequence which contains at least 160 amino acids which is substantially homologous to the C-terminal portion of the CS protein and may be devoid of the end amino acids from the C-terminal of the CS protein, for example the last 10 to 12 amino acids. The hybrid protein may be in the form of mixed lipoprotein particles, for example with HBsAg.

In particular there is provided a hybrid protein as disclosed in WO 93/10152, designated therein as "RTS*" but referred to herein as "RTS", which may be in the form of mixed lipoprotein particles with HBsAg, herein designated RTS,S. The ratio of hybrid protein:S antigen in these mixed particles is for example 1:4.

The hybrid protein designated "RTS" herein was generated using the CS protein gene sequence from *P*. *falciparum NF54* (clone 3D7; Caspers et al. 1989) and comprises substantially the entire region 207 to 395 of the CS protein from *P*. *falciparum* NF54. The portion of the NF54 (3D7) CS protein sequence that is included in RTS is the following sequence of 189 amino acids:
DPNANPNANP NANPNANPNA NPNANPNANP NANPNANPNA NPNANPNANP NANPNANPNA NPNANPNANP NANPNKNNQG NGQGHNMPND PNRNVDENAN ANSAVKNNNN EEPSDKHIKE YLNKIQNSLS TEWSPCSVTC GNGIQVRIKP GSANKPKDEL DYANDIEKKI CKMEKCSSVF NVVNSSIGL (SEQ ID NO:1).

In particular RTS is:
- A methionine residue encoded by nucleotides 1059-1061 derived from the *Sacchromyces cerevisiae* TDH3 gene sequence (nucleotides 1-1058 in this reading frame make up the TDH3 promoter itself). (Musti et al. 1983).
- Three amino acids: Met Ala Pro, derived from a nucleotide sequence (1062-1070) created by the cloning procedure used to construct the hybrid gene).
- A stretch of 189 amino acids (given above, SEQ ID NO:1) encoded by 1071-1637 representing amino acids 207 to 395 of the CS protein of *P*. *falciparum* strain NF54 (clone 3D7; Caspers et al. 1989).
- An amino acid (Gly) encoded by nucleotides 1638 to 1640, created by the cloning procedure used to construct the hybrid gene.
- Four amino acids, Pro Val Thr Asn, encoded by nucleotides 1641 to 1652, and representing the four carboxy terminal residues of the hepatitis B virus (adw serotype) preS2 protein (Valenzuela et al. 1979).
- A stretch of 226 amino acids, encoded by nucleotides 1653 to 2330, and specifying the S protein of hepatitis B virus (adw serotype) (Valenzuela et al. 1979).

RTS may be in the form of mixed particles, RTS,S, where the ratio of RTS:S is for example 1:4.

Although the invention is by no means limited to malarial antigens, the invention will be explained in great detail using viral vectors encoding a malarial antigen in combination with an adjuvated proteinaceous malarial antigen. Those of skill in the art will be able to modify the general teaching provided herein by using different antigenic inserts and corresponding proteinaceous antigens from other pathogenic agents, including parasites, bacteria, viruses, yeasts, or even self-antigens, including, but not limited to, tumor antigens (e.g., PSA, gp100, CEA, MUC1, Her2/neu) and the like).

The present invention relates to a replication-defective recombinant adenoviral vector comprising a heterologous nucleic acid sequence encoding an antigen of *Plasmodium falciparum.* In a preferred embodiment said viral vector is an adenovirus derived from a serotype selected from the group consisting of: Ad11, Ad24, Ad26, Ad34, Ad35, Ad48, Ad49 and Ad50. The reason for this selection of human adenoviruses is because the use of adenoviruses in general as vaccine vectors is typically hampered by the fact that humans are infected regularly with wild type adenoviruses, which cause mild or inapparent diseases such as the common cold. The immune responses raised during such an infection with a parental wild-type serotype can negatively impact the efficacy of the recombinant adenovirus serotype when used as a subsequent recombinant vaccine vector, such as a vaccine against malaria in which adenoviruses are applied. The spread of the different adenovirus serotypes in the human worldwide population differs from one geopgraphic area to the other. Generally, the preferred serotypes encounter a low neutralizing activity in hosts in most parts of the world, as outlined in several reports in the art.

The inventors of the present invention have now made a novel combination between a recombinant adenovirus and a purified protein in a sequential vaccination scheme, referred to as a heterologous prime/boost, which scheme makes use of the different immune responses induced by the different components of the prime/boost vaccine. Choice of the recombinant vector is influenced by those that encounter neutralizing activity in a low percentage of the human population in need of the vaccination. Surprisingly, the combination of adenovirus-vectored antigen and adjuvated protein antigen provides a significant improvement in immune responses over those seen using either vaccine alone. The immune enhancement is illustrated by in vitro detection of immune responses given in vivo to rhesus macaques as disclosed herein.

In another embodiment, the recombinant replication-defective adenovirus is a simian adenovirus, such as those isolated from chimpanzee. Examples that are suited include C68 (also known as Pan 9; US 6,083,716) and Pan 5, 6 and 7 (WO 03/046124).

In one particular aspect of the invention the replication-defective recombinant viral vector comprises a nucleic acid sequence coding for the CS protein, or an immunogenic part or fragment thereof. Preferably, said heterologous nucleic acid sequence is codon-optimized for elevated expression in a mammal, preferably a human. Codon-optimization is based on the required amino acid content, the general optimal codon usage in the mammal of interest and a number of aspects that should be avoided to ensure proper expression. Such aspects may be splice donor or -acceptor sites, stop codons, Chi-sites, poly(A) stretches, GC- and AT-rich sequences, internal TATA boxes, etcetera. Methods of codon optimization for mammalian hosts are well known to the skilled person and can be found in several places in molecular biology literature.

In a preferred embodiment, the invention relates to a replication-defective recombinant adenoviral vector according to the invention, wherein the adenine plus thymine content in said heterologous nucleic acid, as compared to the cytosine plus guanine content, is less than 87%, preferably less than 80%, more preferably less than 59% and most preferably equal to approximately 45%. The invention provides, in one embodiment a replication-defective recombinant adenoviral vector, wherein the CS protein is any one of the CS proteins as disclosed in WO 2004/055187, most preferably the CS protein from *P.falciparum* or an immunogenic fragment thereof.

The production of recombinant adenoviral vectors harboring heterologous genes is well-known in the art and typically involves the use of a packaging cell line, adapter constructs and cosmids and deletion of at least a functional part of the E1 region from the adenoviral genome (see also below for packaging systems and preferred cell lines).

The invention also relates to kits comprising as components on the one hand a recombinant adenoviral vector that encounters low neutralizing activity in the host and on the other hand a purified protein, wherein it is preferred that the purified protein is provided in an admixture with an adjuvant. A preferred adjuvant is QS21 and 3D-MPL, preferably in a formulation with cholesterol-containing liposomes. The components are used in a heterologous prime/boost vaccine delivery strategy in which it is preferred to first administer the recombinant adenoviral vector as a priming agent and then the purified protein as a boosting agent, which boost may be repeated more than once. The components are typically held in pharmaceutically acceptable carriers. Pharmaceutically acceptable carriers are well known in the art and used extensively in a wide range of therapeutic products. Preferably, carriers are applied that work well in vaccines. More preferred are vaccines further comprising an adjuvant. Adjuvants are known in the art to further increase the immune response to an applied antigen. The invention also relates to the use of a kit according to the invention in the therapeutic, prophylactic or diagnostic treatment of malaria.

The present invention relates to a method of treating a mammal for a malaria infection or preventing a malaria infection in a mammal, said method comprising (in either order, or simultaneously) the steps of administering a recombinant adenovirus carrying an antigen of *P.falciparum;* and administering at least one purified *P.falciparum* protein, said protein admixed with an adjuvant. Preferably the recombinant adenovirus is selected from the group consisting of Ad11, Ad24, Ad26, Ad34, Ad35, Ad48, Ad49 and Ad50, while it is also preferred that the recombinant adenovirus harbors the gene encoding the CS protein, or an immunogenic fragment thereof. The preferred purified protein that is used in combination with the recombinant adenovirus is RTS,S, while a preferred adjuvant is QS21 and 3D-MPL, preferably in a formulation with cholesterol-containing liposomes.

The driving force behind the development of the immune responses is cytokines, a number of identified protein messengers that serve to help the cells of the immune system and steer the eventual immune response to either a Th1 or Th2 response. Thus, high levels of Thl-type cytokines tend to favor the induction of cell mediated immune responses to the given antigen, while high levels of Th2-type cytokines tend to favor the induction of humoral immune responses to the antigen. It is important to remember that the distinction of Th1 and Th2-type immune responses is not absolute. In reality, an individual will support an immune response that is described as being predominantly Th1 or predominantly Th2. However, it is often convenient to consider the families of cytokines in terms of that described in murine CD4+ T cell clones by Mosmann and Coffman (1989). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of IL-4, IL-5, IL-6, IL-10 and tumour necrosis factor- (TNF-ss).

Suitable adjuvants for use in the invention include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, polyphosphazenes, or montanide liposomes.

In the formulation of vaccines for use in the invention, in the context of the adenovirus vector, an adjuvant may or may not be administered. In the case of the protein component of the combination, the adjuvant composition may be selected to induce a preferential Th1 response. Moreover, other responses, including other humoral responses, may also be induced.

Certain vaccine adjuvants are particularly suited to the stimulation of either Th1 or Th2-type cytokine responses. Traditionally, the best indicators of the Th1: Th2 balance of the immune response after a vaccination or infection includes direct measurement of the production of Th1 or Th2 cytokines by T lymphocytes in vitro after restimulation with antigen, and/or the measurement of the IgG1: IgG2a ratio of antigen specific antibody responses. Thus, a Th1-type adjuvant is one, which stimulates isolated T-cell populations to produce high levels of Th1-type cytokines when re-stimulated with antigen in vitro, and induces antigen specific immunoglobulin responses associated with Th1-type isotype. For example, Th1-type immunostimulants which may be formulated to produce adjuvants suitable for use in the present invention may include Monophosphoryl lipid A, in particular 3-de-O-acylated monophosphoryl lipid A (3D-MPL). 3D-MPL is a well-known adjuvant manufactured by Ribi Immunochem, Montana. Chemically it is often supplied as a mixture of 3-de-O-acylated monophosphoryl lipid A with either 4,5, or 6 acylated chains. It can be purified and prepared by the methods taught in GB 2122204B, which reference also discloses the preparation of diphosphoryl lipid A, and 3- O-deacylated variants thereof. Other purified and synthetic lipopolysaccharides have been described (US Pat. 6,005,099, EP 0729473 B1, EP 0549074 B1). In one embodiment, 3D-MPL is in the form of a particulate formulation having a small particle size less than 0.2 µm in diameter, and its method of manufacture is disclosed in EP 0689454.

Saponins are another example of Th1 immunostimulants that may be used with the invention. Saponins are well known adjuvants. For example, Quil A (derived from the bark of the South American tree Quillaja Saponaria Molina), and fractions thereof, are described in US Pat. 5,057,540, and EP 0362279 B1. The haemolytic saponins QS21 and QS17 (HPLC purified fractions of Quil A) have been described as potent systemic adjuvants, and the method of their production is disclosed in US Pat. 5,057,540 and EP 0362279 B1. Also described in these references is the use of QS7 (a non-haemolytic fraction of Quil-A), which acts as a potent adjuvant for systemic vaccines. Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Yet another example of an immunostimulant is an immunostimulatory oligonucleotide containing unmethylated CpG dinucleotides ("CpG"). CpG is an abbreviation for cytosine-guanosine dinucleotide motifs present in DNA. CpG is known in the art as being an adjuvant when administered by both systemic and mucosal routes (WO 96/02555, EP 0468520). Historically, it was observed that the DNA fraction of bacillus Calmette-Guerin (BCG) could exert an anti-tumor effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the CG motif is not methylated, but other unmethylated CpG sequences are known to be immunostimulatory and may be used in the present invention.

In certain combinations of the six nucleotides, a palindromic sequence may be present. Several of these motifs, either as repeats of one motif or a combination of different motifs, can be present in the same oligonucleotide. The presence of one or more of these immunostimulatory sequences containing oligonucleotides can activate various immune subsets, including natural killer cells (which produce interferon y and have cytolytic activity) and macrophages. Other unmethylated CpG containing sequences not having this consensus sequence have also now been shown to be immunomodulatory. When formulated into vaccines, CpG is generally administered in free solution together with free antigen (WO 96/02555, 68) or covalently conjugated to an antigen (WO 98/16247), or formulated with a carrier such as aluminium hydroxide (Hepatitis surface antigen).

Such immunostimulants as described above may be formulated together with carriers, such as, for example, liposomes, oil in water emulsions, and or metallic salts, including aluminium salts (such as aluminium hydroxide). For example, 3D-MPL may be formulated with aluminium hydroxide (EP 0689454) or oil in water emulsions (WO 95/17210); QS21 may be advantageously formulated with cholesterol containing liposomes (WO 96/33739), oil in water emulsions (WO 95/17210) or alum (WO 98/15287); CpG may be formulated with alum or with other cationic carriers.

Combinations of immunostimulants may also be used, such as a combination of a monophosphoryl lipid A and a saponin derivative (WO 94/00153; WO 95/17210; WO 96/33739; WO 98/56414; WO 98/05355; WO 99/12565; WO 99/11241) or a combination of QS21 and 3D-MPL as disclosed in WO 94/00153. Alternatively, a combination of CpG plus a saponin such as QS21 may also be used in the present invention. Thus, suitable adjuvant systems include, for example, a combination of monophosphoryl lipid A, such as 3D-MPL, together with an aluminium salt. Another embodiment combines a monophosphoryl lipid A and a saponin derivative, such as the combination of QS21 and 3D-MPL as disclosed in WO 94/00153, or a less reactogenic composition where the QS21 is quenched in cholesterol containing liposomes (DQ) as disclosed in WO 96/33739. Yet another adjuvant formulation involving QS21,3D-MPL and tocopherol in an oil in water emulsion is described in WO 95/17210. In another embodiment, CpG oligonucleotides are used alone or together with an aluminium salt.

A suitable adjuvant for use in the present invention is a preferential Th1 stimulating adjuvant, for example an adjuvant comprising a saponin such as QS21 or a monophosphoryl lipid A derivative such as 3D-MPL, or an adjuvant comprising both of these optionally together with cholterol-containing liposomes. A combination of QS21 and 3D-MPL in a formulation with cholesterol-containing liposomes is described for example in WO 96/33739

The advantages of the present invention are multi-fold. Recombinant viruses, such as recombinant adenoviruses, can be produced to very high titers using cells that are considered safe, and that can grow in suspension to very high volumes, using medium that does not contain any animal- or human derived components. Also, it is known that recombinant adenoviruses elicit a dramatic immune response against the protein encoded by the heterologous nucleic acid sequence in the adenoviral genome. The present invention combines these features in a vector harboring the circumsporozoite gene of *P.falciparum* with the use of adjuvated protein to boost responses. Moreover, the gene has been codon-optimized to give an expression level that is suitable for giving a proper immune response in humans. The present invention provides a vaccine against malaria infections, making use of adenoviruses that do not encounter high titers of neutralizing antibodies. Highly preferred adenoviruses for this purpose are serotype 11 and 35 (Ad11 and Ad35, see WO 00/70071 and WO 02/40665).

The nucleic acid content between the malaria-causing pathogen, such as *P.falciparum* and the host of interest, such as *Homo sapiens* is very different. The invention provides codon-optimised nucleic acids providing higher expression levels in mammals, such as humans.

The use of different entities for prime/boost regimens as disclosed herein provides a vaccine method that provides for proper immune responses of both cellular and humoral arms of the immune system. It involves CD8+ T cells, CD4+ T cells and antibodies. Neither of these vaccines alone establishes a sustainable immune response that invokes optimal levels of antigen-specific CD8+ T cells, CD4+ T cells and antibodies. Moreover, the order in which the different components are administered may alter these immune responses and may give rise to different periods of possible protection against future infections. The methods and kits of the present invention enable one to elicit an immune response that deals with all the different stages of the parasite's life cycle in humans, from free circulating sporozoites and merozoites to infected hepatocytes and RBC's. Moreover, it provides a sustained protection against malaria infections over a prolonged period of time.

In a preferred embodiment, the invention relates to the use of recombinant adenoviruses that are replication defective through removal of at least part of the E1 region in the adenoviral genome, since the E1 region is required for replication-, transcription-, translation- and packaging processes of newly made adenoviruses. E1 deleted vectors are generally produced on cell lines that complement for the deleted E1 functions. Such cell lines and the use thereof for the production of recombinant viruses have been described extensively and are well known in the art. Preferably, PER.C6^{©} cells, as represented by the cells deposited under ECACC no. 96022940 at the European Collection of Animal Cell Cultures (ECACC) at the Centre for Applied Microbiology and Research (CAMR, UK), or derivatives thereof are being used to prevent the production of replication competent adenoviruses (rca). In another preferred embodiment, cells are being applied that support the growth of recombinant adenoviruses other than those derived for adenovirus serotype 5 (Ad5). Reference is made to publications WO 97/00326, WO 01/05945, WO 01/07571, WO 00/70071, WO 02/40665 and WO 99/55132, for methods and means to obtain rca-free adenoviral stocks for Ad5 as well as for other adenovirus serotypes, such as recombinant replication-defective Ad35 which may be produced on HER cells immortalized with E1 from Ad35, or on PER.C6^{©} cells that further comprises E1 genes from Ad35 to provide proper complementation of B-type adenoviruses.

It must be noted here that in the published documents WO 00/03029, WO 02/24730, WO 00/70071 and WO 02/40665, Ad50 was mistakenly named Ad51. The Ad51 serotype that was referred to in the mentioned publications is the same as serotype Ad50 in a publication by De Jong et al. (1999), wherein it was denoted as a B-group adenovirus. For the sake of clarity, Ad50 as used herein, is the B-group Ad50 serotype as mentioned by De Jong et al. (1999).

The vaccines of the present invention are typically used in prime/boost settings, for example Ad/protein; protein/Ad; protein/Ad/Ad; Ad/protein/Ad; Ad/Ad/protein, Ad/protein/protein/protein, Ad/protein/viral vector/protein, etc, etc. It may be envisioned that a combination with yet another kind of vaccine (such as naked DNA or a recombinant viral vector different from adenovirus) may be applied in combination with the prime/boost agents of the present invention. Additional malarial antigens or (poly)peptides may also be used.

A sequence is 'derived' as used herein if a nucleic acid can be obtained through direct cloning from wild-type sequences obtained from wild-type viruses, while they can for instance also be obtained through PCR by using different pieces of DNA as a template. This means also that such sequences may be in the wild-type form as well as in altered form. Another option for reaching the same result is through combining synthetic DNA. It is to be understood that 'derived' does not exclusively mean a direct cloning of the wild type DNA. A person skilled in the art will also be aware of the possibilities of molecular biology to obtain mutant forms of a certain piece of nucleic acid. The terms 'functional part, derivative and/or analogue thereof' are to be understood as equivalents of the nucleic acid sequence they are related to. A person skilled in the art will appreciate the fact that certain deletions, swaps, (point) mutations, additions, etcetera may still result in a nucleic acid sequence that has a similar function as the original nucleic acid sequence, and should produce a similar or even identical polypeptide once translated. It is therefore to be understood that such alterations that do not significantly alter the functionality of the nucleic acid sequences are within the scope of the present invention. If a certain adenoviral vector is derived from a certain adenoviral serotype of choice, it is also to be understood that the final product may be obtained through indirect ways, such as direct cloning and synthesizing certain pieces of genomic DNA, using methodology known in the art. Certain deletions, mutations and other alterations of the genomic content that do not alter the specific aspects of the invention are still considered to be part of the invention. Examples of such alterations are for instance deletions in the viral backbone to enable the cloning of larger pieces of heterologous nucleic acids. Examples of such mutations are for instance E3 deletions or deletions and/or alterations in the regions coding for the E2 and/or E4 proteins of adenovirus. Such changes applied to the adenoviral backbone are known in the art and often applied, since space is a limiting factor for adenovirus to be packaged; this is a major reason to delete certain parts of the adenoviral genome. Other reasons for altering the E2, E3 and/or E4 regions of the genome may be related to stability or integrity of the adenoviral vector, as for instance described in WO 03/104467 and WO 2004/001032). These applications relate amongst others to the use of an E4 or f6 gene from a serotype from one subgroup in the backbone of an adenovirus from another subgroup, to ensure compatibility between the E4 or f6 activity and the E1B-55K activity during replication and packaging in a packaging cell line. They further relate to the use of a proper functioning pIX promoter for obtaining higher pIX expression levels and a more stable recombinant adenoviral vector.

'Replication defective' as used herein means that the viral vectors do not replicate in non-complementing cells. In complementing cells, the functions required for replication, and thus production of the viral vector, are provided by the complementing cell. The replication defective viral vectors of the present invention do not harbor all elements enabling replication in any host cell other than a complementing cell.

'Heterologous' as used herein in conjunction with nucleic acids means that the nucleic acid sequence derives from a different original source than the wild type versions of the viral vectors in which the heterologous nucleic acid is cloned. For instance in the case of adenoviruses, the heterologous nucleic acid that is cloned in the replication defective adenoviral vector, is not an adenoviral nucleic acid sequence, but comes from some other pathogenic agent of interest.

'Heterologous' as used herein in conjunction with prime-boost vaccine strategies means that two or more separate components, exemplified by one recombinant non-replicative adenovirus vector and one adjuvated protein used in deliberate combination, rather than one component being administered several times, as is usual in the industry thus far.

'Antigen' as used herein means any antigen derived from a source that elicits an immune response in a host to which the determinant is delivered (administered). The antigen may be from an external source, e.g. a pathogen, a parasite, or even be a self-antigen. Examples of antigens of *Plasmodium* that can be delivered by using the replication defective recombinant viruses of the present invention are the circumsporozoite protein (CS), the SE36 polypeptide, the merezoite surface protein-1 19 kDa C-terminal polypeptide (MSP-1p19), MSP-1, MSP-1p42, Apical Merozoite Antigen-1 (AMA-1), Liver Stage Antigen 1 (LSA-1) or Liver Stage Antigen-3 (LSA-3), or a fragment of any of the aforementioned. In a preferred aspect the invention relates to the circumsporozoite (CS) protein from *P.falciparum.*

'Codon-optimized' as used herein means that the nucleic acid content of a sequence has been altered to support sufficiently high expression levels of the protein of interest in a host of interest to which the gene encoding said protein is delivered. Sufficiently high expression levels in this context means that the protein levels should be high enough to elicit an immune response in the host in order to protect against infection or against disease. It is known in the art that some vaccines give an immune response in humans, through which approximately 60% of the vaccinated individuals is protected against illnesses induced by subsequent challenges with the pathogen (e.g., sporozoites). Therefore the expression levels are considered to be sufficient if 60% or more of the treated individuals is protected against subsequent infections. It is believed that with the combinations of adenoviral aspects that can be applied and the choice of antigen as disclosed herein, such percentages may be reached. Preferably, 85% of the individuals are protected, while it is most preferred to have protection to a subsequent challenge in more than 90% of the vaccinated hosts. The nucleic acids disclosed in the present invention are codon-optimized for expression in humans. According to Narum et al. (2001), the content of adenine plus thymine (A+T) in DNA of *Homo sapiens* is approximately 59%, as compared to the percentage cytosine plus guanine (C+G). The adenine plus thymine content in *P.falciparum* overall is approximately 80%. The adenine plus thymine content in the CS gene of *P.falciparum* is approximately 87%. To obtain sufficient protection it is believed to be necessary to improve production levels in the host. One way to achieve this is to adjust codon usage to maintain the same ultimate amino acid sequence, but use codon sequences more typical of mammalian expression. For this, the replication-defective recombinant viral vectors according to the invention have an adenine plus thymine content in the heterologous nucleic acids of the present invention of less than 87%, preferably less than 80%, and more preferably less than or equal to approximately 59%. Based on codon-usage in human and the amino acid content of the CS genes of *P.falciparum* and yoelii, the percentages of the codon-optimized genes were even lower, reaching approximately 45% for the amino acid content as disclosed by the present invention. Therefore, as far as the CS genes are concerned it is preferred to have an adenine plus thymine content of approximately 45%. It is to be understood, that if another species than humans is to be treated, which may have a different adenine plus thymine concentration (less or more than 59%), and/or a different codon usage, that the genes encoding the CS proteins of the present invention may be adjusted to fit the required content and give rise to suitable expression levels for that particular host. Of course, it cannot be excluded either, that slight changes in content may result in slight expression level changes in different geographical areas around the world. It is also to be understood that slight changes in the number of repeats included in the amino acid sequence of the proteins, that percentages may differ accordingly. Other antigens of interest may be similarly modified. All these adjusted contents are part of the present invention.

The protein designated RTS,S is a fusion protein consisting of the C-terminal half of the *P. falciparum* CS protein (17 of the central 41 NANP-repeats plus most of the C-terminal portion) expressed as a fusion protein with the Hepatitis B Surface antigen.

One of the distinct advantages offered by the replication-incompetent adenoviral vectors is the minor pathogenicity of the parental viruses and the documented lack of significant disease caused by these vectors in any individual, including those who are immunosupressed. Work in the mouse model of malaria, *P. yoelii,* indicated that recombinant adenovirus constructs expressing the CS protein not only engender outstanding cellular immune responses, they provide excellent protection against infection. Therefore, in an effort to improve the intensity of the T cell response and the longevity of the overall immune response to CS, the inventors of the present invention decided to combine an adenoviral approach with the recombinant protein approach in a novel heterologous prime-boost strategy.

Unfortunately, the mouse is not the ideal model for predicting responses in humans. This is particularly true for Adenovirus 35 (Ad35). The standard replication-incompetent vector is Adenovirus 5 (Ad5), which has demonstrated some problems with optimizing its vector capabilities due to the widespread endemnicity of this virus and the fact that a substantial proportion of most global human populations have pre-existing immunity to the parental virus. Ad35 has the potential to demonstrate enhanced utility as a vaccine vector. The availability of both Ad5 and Ad35 CSP-bearing constructs allowed evaluation of two sequential heterologous adenoviral immunizations with differing constructs specifically for the question of CS immunity.

Dendritic cells (DC) are the most potent antigen-presenting cells in the body, and the fact that both Ad5 and Ad35 target to human and rhesus DC is one of the aspects of their biology that makes them such excellent vaccine vectors. However, only Ad5 efficiently infects murine DC; Ad35 only reliably infects primate DC. Thus, although basic potency questions about Ad35 constructs can be answered in small animal models, actual immunogenicity questions involving Ad35 can only be asked in non-human primates.

The inventors decided to examine the prime-boost combinations of RTS,S with adenoviral vectors containing the CS gene to determine if the anti-malarial cellular and/or humoral responses would be an improvement upon the responses seen to RTS,S alone. In addition, a regimen for two doses of adenovirus vaccine alone was optimized.

### EXAMPLE

### Heterologous prime/boost vaccination using recombinant adenoviral vectors and purified adjuvated protein in rhesus monkeys.

The objectives of the experiment were to evaluate RTS,S followed by Ad35, and Ad35 followed by RTS,S, in a direct comparison with a standard three-dose RTS,S immunization regimen and a standard two-dose Ad35 regimen. A secondary objective was to optimize the two-dose adenovirus regimen. The serologic and cellular immune responses during and after several different regimens of these constructs in combination were studied.

The rhesus monkey (*Macaca mulatta*) makes an excellent model for the human immune response because of its much closer phylogenetic relationship. MHC Class II alleles are particularly well conserved; the generation of some shared alleles is estimated at 25 million years ago, predating the speciation of human and rhesus. Thus, there is similar epitope usage in presentation of antigen to Th cells, which greatly enhances the predictive value of the model. More importantly, the rhesus monkey model has in the past been proven to be highly predictive of the human immunogenicity responses both for malaria antigens and for HIV, another human disease for which the development of a vaccine has been hindered by the complexity of the immune response.

Preliminary experiments have already been performed in mice with adenoviral-CS constructs of the mouse malaria *P*. *yoelii* that demonstrated excellent immunogenicity and protective efficacy. However, the long history of unsuccessful attempts to directly extrapolate from the mouse malaria model to humans in the quest for development of vaccines for falciparum malaria mandates an intermediate step in a non-human primate model. The rhesus macaque represents the best choice of species because of the extensive database of prior information on these vaccines in this species, because of the phylogenetic proximity to humans, because their size permits blood samples of sufficient volume to ensure adequate assessment of immune responses, and because reagents and assays exist that are already optimized for this species and thus do not require ancillary protocols and many years to develop. Additionally, the adenovirus 35 constructs can only be appropriately tested in non-human primates, because of the inability of this virus to efficiently invade the dendritic cells of other mammals.

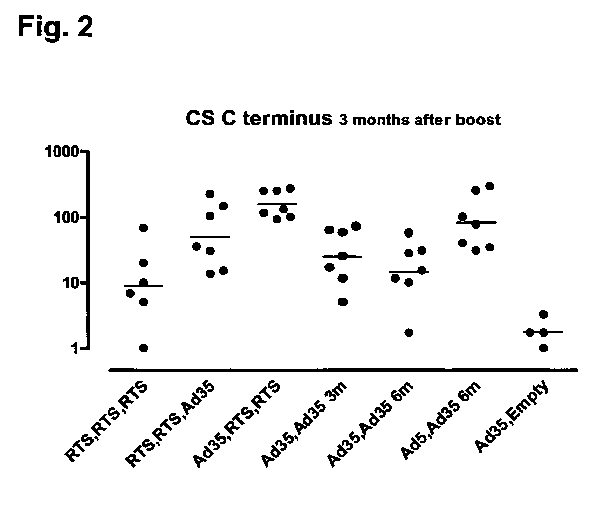

The constructs and the production of the recombinant, replication-defective adenoviruses harboring the *P.falciparum* CS encoding gene (Ad5CS and Ad35CS) used in this study have been described in great detail in the examples of WO 2004/055187 (clone 02-659; see fig. 2 therein). Briefly, these adenovectors comprise a heterologous gene encoding for the CS protein with an amino acid sequence that is similar to the CS protein of the NF54 strain, 3D7 clone, having amongst others, an N-terminal signal sequence, 27 NANP repeats, a cluster of 3 NVDP repeats and one separate NVDP repeat, the universal epitope (Lockyer et al. 1989; Zevering et al. 1994; Nardin et al. 2001), and a deletion of the last 14 amino acids (at the C-terminus). The difference with the protein of RTS,S is that RTS,S lacks the N-terminal signal sequence, and a large portion of the repeat region, as well as most of the C-terminally located GPI anchor signal sequence which is also absent in the adenoviral constructs.

The experiment was a randomized, blinded safety and immunogenicity study of various combinations and timing strategies for optimization of prime-boost strategies of RTS,S with Ad5 and Ad35 CS-bearing constructs (Ad5CS and Ad35CS) and for optimization of Ad5CS and Ad35CS alone. The previous best regimen against which the new strategies were compared were three intramuscular doses of 50 µg of RTS,S with adjuvant given at 0, 1, and 3 months. This was Group 1, the Positive Control group. All groups are outlined in Table 1A. In all cases the adjuvant was made up of 50 µg of 3D-MPL, 50 µg QS21, in a formulation with cholesterol-containing liposomes as described in WO 96/33739.

Group 2 received two doses of RTS,S/Adjuvant at 0 and 1 month followed by one dose of Ad35CS at 3 months. Group 3 received one dose of Ad35CS at month 0 followed by two doses of RTS,S/Adjuvant at 1 and 3 months.

Groups 4, 5, and 6 only received adenoviral constructs. Prior experience with two doses of adenovirus 5 constructs in different diseases has indicated that optimal serologic and cellular immune responses are obtained when the interval between immunizations is at least 6 months. Because of the necessity to evaluate Ad35 constructs in humans or non-human primates, the optimal time between doses for this vector was not yet established. Thus, Group 4 received two doses of Ad35CS on a 0, 3 month schedule (for a direct control to the protein groups), and Group 5 got two doses on a 0, 6 month schedule. In order to evaluate the question of whether two doses of the same adenovirus construct were inferior to alternation of constructs for the CS protein, Group 5 was compared with Group 6, which received Ad5CS followed by Ad35CS on the 0, 6 month schedule.

Finally, control Group 7 got two doses of plain (no malaria gene insert) Ad35 at 0 and 3 months to serve as an immunization control group for immunogenicity assessments.

Injection sites were clipped and clearly marked to facilitate observation of vaccine reactogenicity. Additionally, the animals were sedated and the injection site was directly examined for signs of induration, swelling, heat, redness, or other abnormality at 24, 48, and 72 h and at 7 and 14 days post injection. Although signs of systemic toxicity were not expected, the animals were also sedated and examined at these time points for lymphadenopathy, cellulitis, abscessation, arthritis, anorexia, and weight loss, and their hematologic and clinical chemistry values were monitored for alterations. Blood was drawn at the time of injection and at 24, 48, 72 h and 7 and 14 days after each injection for complete blood count (CBC) and for a panel of clinical chemistry assays that included (but not necessarily limited to) determinations of BUN, creatinine, AST, ALT, GGT, and CK. Fecal samples to confirm the absence of non-replicative vector shedding were collected and saved at -70°C on each of Days 0-10 for each adenovirus injection, for subsequent adenovirus testing.

1-3 mls of serum was collected at the time of and 1, 2, and 4 weeks after every injection, and at least once monthly thereafter to determine the nature and magnitude of the antibody response to CS R32 (the repeat region of the CS protein used to develop the standardized ELISA assay to the CS protein, see below) by ELISA. Serum samples were stored at -70°C until use, and the samples were batch processed near the end of the experiment to minimize intra-assay variability. Volumes of serum collected were adequate so that for each adenovirus injection, 0.5-1.0 ml of serum from Day 0, 1, 7 and 14, and at least every 4 weeks thereafter can be used for anti-adenovirus antibody titer determination. Large volumes (20-40 ml) of EDTA- or heparin-anti-coagulated blood was collected for cell harvests prior to the first immunization, 4 weeks after the second immunization (if volume demands permit), 4 weeks after the third immunization, and 6 months after the third immunization. Peripheral blood mononuclear cells (PBMC) were concentrated from these samples using standard methods of density centrifugation separation. Although cell yields can be highly variable from one individual animal to another, in general the larger the volume of the sample, the greater the number of recovered cells. Because it is impossible to predict the exact cell recovery, it is preferred to take a larger sample where possible so that enough cells were obtained to repeat assays for purposes of statistical validity. Cells were frozen to permit batch processing at a later time point and thereby improve quality control. Cells were frozen in autologous serum with 10% DMSO at a controlled temperature reduction rate and stored in vapor-phase liquid nitrogen for at least a week before use.

From the larger animals whose CBC data indicated it would be well tolerated, an additional sample for cell harvest was collected after the prime, but before the boost. Since there were 14 monkeys (two groups) that received two doses of RTS,S/Adjuant and 14 monkeys that received a single dose of Ad35CS prior to the 8^{th} week, it was expected to sample at least half and thereby maintain statistical significance. Cell harvests would occur no sooner than 4 weeks after an injection. Since the groups getting only adenovirus constructs received only two injections, and thus have a less demanding bleeding schedule than the monkeys receiving three injections, a cell harvest intermediate between the two injections was expected to pose no hardship.

Analyses of cellular immune responses included short-term ELISPOT assays for quantitation of antigen-specific IFN-γ producing cells. Flow cytometric analysis of antigen-stimulated cells cannot only confirm data gathered in ELISPOT analyses, but provides additional information about the phenotype of the antigen-specific cells that are responding. Thus, determination of the antigen-specific CD8+ IFN-γ secreting subset by intracellular staining and flow cytometry is also investigated.

Additional assays that are performed include bulk ELISpot analyses for additional cytokines, intracellular staining for T cell subset enumeration of additional cytokines, other flow-cytometric-based assays for quantitation of antigen-specific T cell subset cytokine production, and quantitative RT-PCR for correlation with the other methods.

Monkeys were divided into groups evenly matched for age, sex, weight, and geographical origin, and groups were then randomized. All clinical assessments and safety endpoint determinations were determined without knowledge of the group assignment of the monkeys. Similarly, all immunological assays were performed without prior knowledge of the groups to which the individual samples belong. The exception to this blinding policy were the animals receiving immunizations on a 0 and 6 month schedule as opposed to 0, 1, and 3; however, blinding was maintained as to the specific injection being given.

A group size of seven animals per test group (and four in the control group) is ideal to minimize group size but to still accurately detect differences between groups, based on prior data from similar, but only distantly related, experiments.

The geometric means of results of ELISA assays were compared parametrically using standard analyses such as Student's t-test, assuming equal variance and two tails, and ANOVA. Results of ELISPOT assays, expressed in spots per 200,000 cells, were treated similarly and were also examined using non-parametric analyses such as the Kruskal-Wallis test. Where intergroup comparisons are required, the Student's t-test, on raw or log-transformed data, is used to determine differences between any pair of groups.

Prior to injection, the hair was clipped and the skin cleaned with 70% rubbing alcohol, and a 2.5-3 cm circle drawn on the skin in indelible ink to facilitate locating the injection site for subsequent palpation and reactogenicity assessment. Injections of RTS,S were mixed with the adjuvant immediately prior to entering the monkey corridor. The final injection volume was 0.5 ml and delivered through a 25-29 gauge needle into the anterior thigh musculature. Adenovirus constructs were prepared as described (WO 2004/055187) in buffered saline and also administered in the same intramuscular location in a final volume of 0.5 ml.

The primary biosample was blood, whether for serum or cells. A bleeding schedule is outlined in Table 1B. The animals' hematologic status was monitored; indicating the capacity of an individual to maintain repeated biosampling or signifying that the planned biosampling schedule be reduced. Every time blood was taken, a complete blood count (CBC) was performed with a Coulter automated blood cell counter (requiring <50 µl uncoagulated blood). The manufacturer's recommended GLP-like guidelines for maintenance and upkeep were performed. Hematocrit, hemoglobin, mean corpuscular volume (MCV), red blood cell (RBC) count, and reticulocyte percentage were followed closely to assure that the animals did not become anemic.

Venous blood was collected from the femoral, saphenous, or cephalic veins using 20-24 gauge needles and either syringes or vacuum tubes. In general, the saphenous or cephalic veins were preferred for blood draws of less than 10 ml, and the femoral veins were preferred to avoid hemolysis and shorten total venipuncture time when volumes of greater than 10 ml were removed.

Peripheral Blood Mononuclear cells (PBMC's) were harvested from the animals before immunization, two weeks after the final immunization, and three months after the final immunization. In this protocol, PBMC's are separated by standard methods of density centrifugation, and cryopreserved in 45% autologous serum (45% saline and 10% DMSO). Briefly, whole blood was layered on Lymphoprep^{®} (Axis-Shield, Oslo, Norway) ficoll-hypaque cell separation medium and centrifuged at 650g for 20 min. The cell layer was removed and washed in two washes of dPBS (BioWhittaker, Walkersville, MD) at 400g for 15 min. Viable cells were counted using a Coulter ACT*10 hemocytometer. Pellets were resuspended to 1x10⁷/ml in 50% dPBS, 50% saline. DMSO was added dropwise to a final 10% volume. Cells were frozen in 0.55 ml aliquots of exactly 5 million cells each by placing in a controlled temperature reduction isoropanol bath in the -70°C freezer overnight, and stored in liquid nitrogen vapor phase until use.

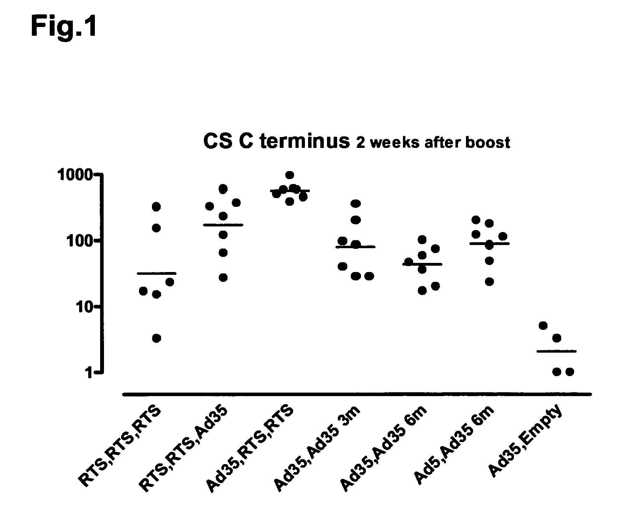

After the last vaccination, interferon gamma (IFN-γ) secreting T cells in blood samples from the different monkeys were identified with the enzyme-linked immunospot (ELISpot) assay after stimulation with whole antigens and C- and N-terminal specific peptide pools (as described in detail below). Results are plotted in figure 1 (2 weeks after the last vaccination) and figure 2 (3 months after the last vaccination), and expressed in table 2 and 4, respectively, as median spot forming units per million cells (SFU); statistical comparison was done using analysis of variance (ANOVA) on log-transformed data. All groups were compared. In case statistical significance was determined a post-hoc analysis can be done for a group-by-group comparison (results not shown).

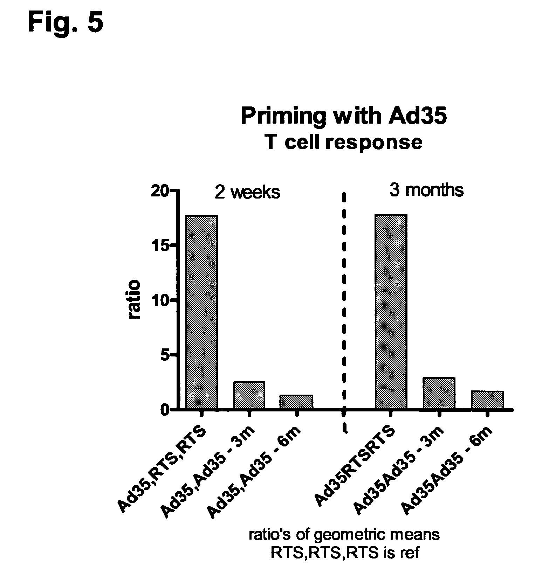

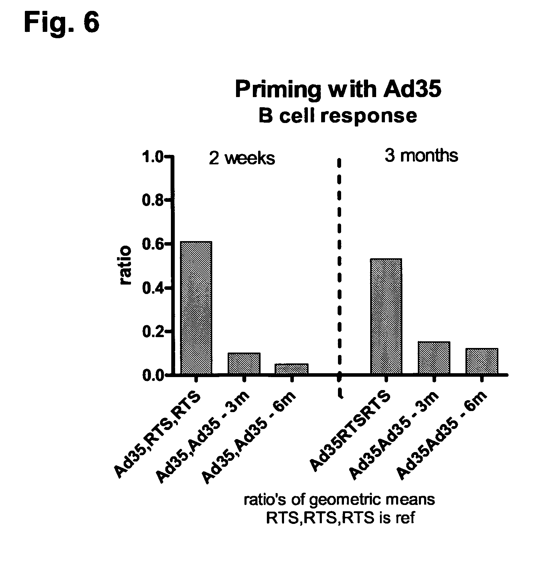

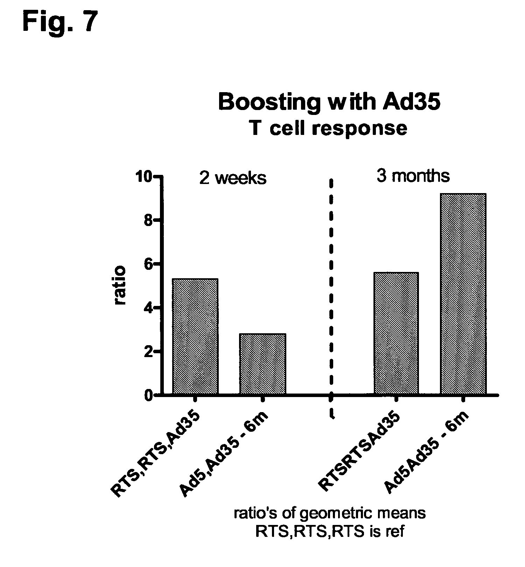

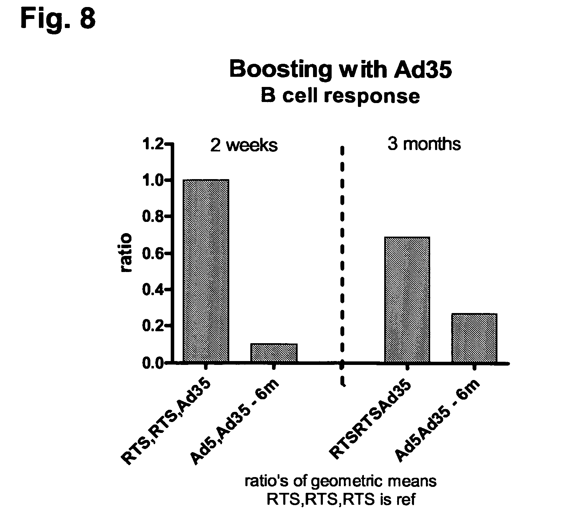

To compare ELISpot results between different treatment strategies, ratios of geo mean titers were calculated for strategies with Ad35 as prime treatment. In these ratios the geo mean titer obtained with treatment with RTS,S alone (at 0, 2, 3 months) was taken as reference treatment (figure 5 and 6, and table 10). Similarly, ratios were also calculated for strategies with Ad35 as boost treatment (figure 7 and 8, and table 11).

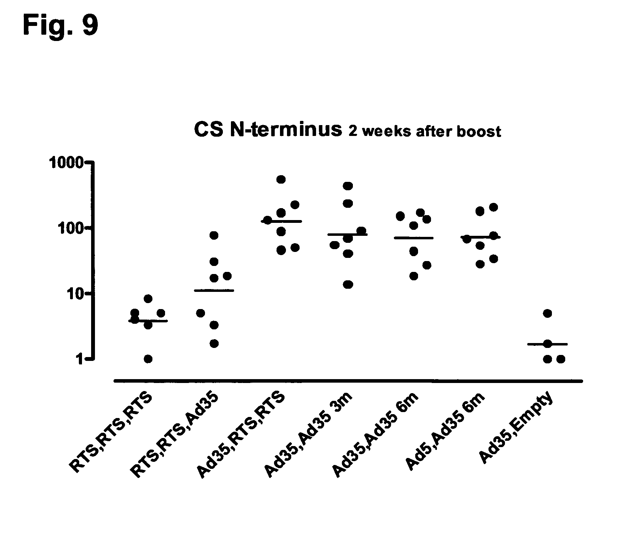

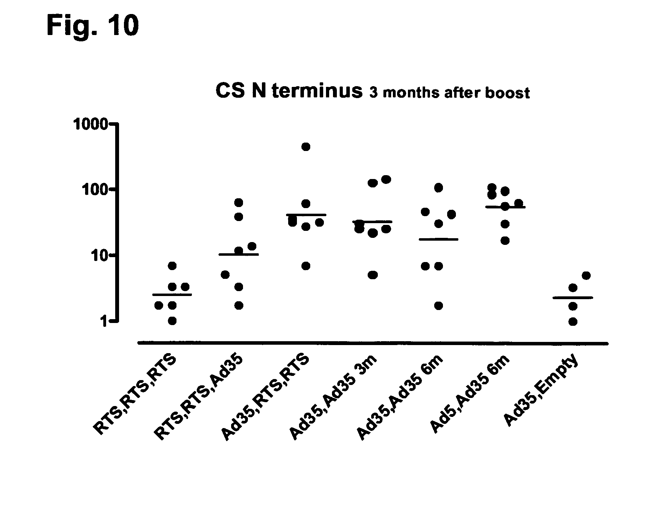

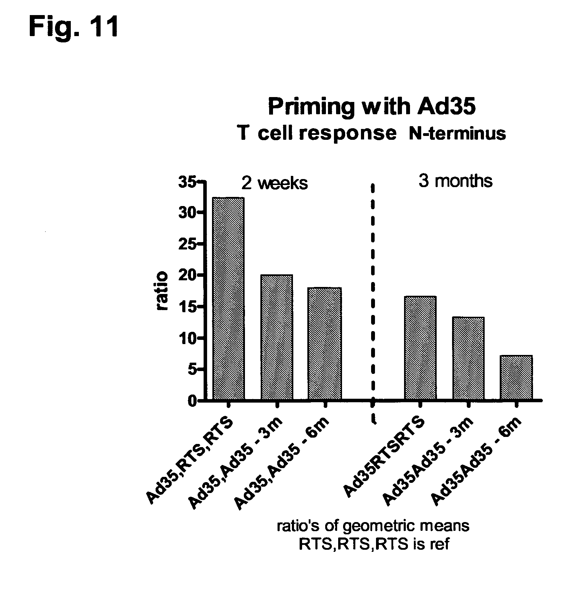

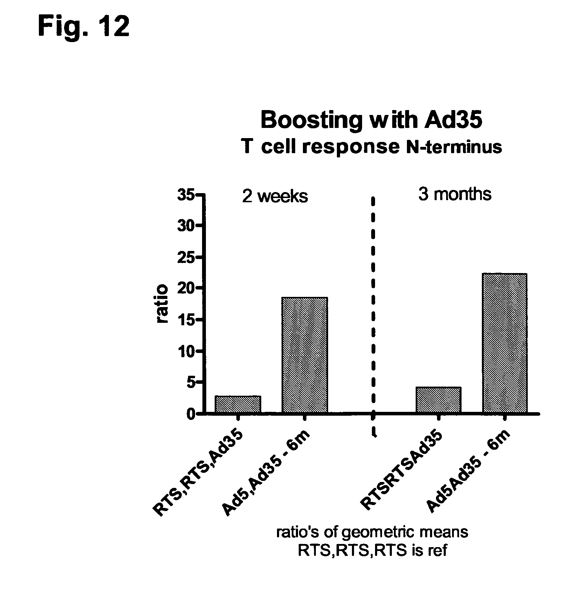

Similar analyses were done for the results of N terminus-specific stimulation T cell ELISpots. Results are plotted in figures 9 and 10, and in tables 12 and 14, respectively. Finally, ratios were calculated and presented in figure 11 (Ad35 priming strategies) and figure 12 (Ad35 boosting strategies), and tables 16 and 17, respectively.

ELISpots were performed on thawed cryopreserved PBMC's in PVDF-bottomed MultiScreen-IP ELISpot plates (Millipore, Bedford, MA) using standard methodology. Sterile technique was strictly adhered to until the cells were removed on Day 2 for final spot development.

Media used: Complete media (cRPMI) was freshly prepared from RPMI-1640 (BioWhittaker, Walkersvile, MD) with the addition of 1:100 penicillin/streptomycin, 1:100 L-glutamine, 1:200 NaHCO₃ (Sigma, St. Louis, MO), 1:100 Non-Essential Amino Acids, 1:100 Pyruvate, and 1:300 2-ME (Gibco). Fetal Calf Serum (FCS, HyClone, Logan, UT), of a lot previously characterized by nonspecific proliferation assay to support good monkey cell growth yet provide little stimulatory background, was added at 10% final volume for cRPMI-10, 20% for cRPMI-20, etc. Media-Plus (M+) was additionally supplemented with anti-monkey CD28 and anti-monkey CD49D antibodies at 1:500 (BD Pharmingen, San Jose, CA).

The following stimulants were prepared fresh at twice the intended final concentration in M+ without added serum:
Con A: Concanavalin A (Sigma) at 2.5 µg/ml (final 1.25 µg/ml) as a positive control for all vials.
CS-C: a pool of 15-mer polypeptides, overlapping by 11 amino acids, covering the C-terminal portion of the PfCS molecule (supplied by GSK, Rixensart, Belgium) at 2.5 µg/ml of each peptide (1.35 µg/ml final).
CS-N: a similar pool of 15mer peptides, overlapping by 11, covering the N-terminus of the PfCS molecule.
RTS,S: purified whole protein complex RTS,S antigen suitable for cell culture (GSK) at 2 µg/ml (1 µg/ml final).
HEF: purified Hepatitis B surface antigen (HbS) whole protein (the "S" component of RTS,S), also suitable for cell culture (GSK) at 23.2 µg/ml (11.6 µg/ml final). HbS-P: a pool of HbS 15mer peptides (GSK) at 2.5 µg/ml each peptide (1.25 µg/ml final).
The negative control was M+ without further supplementation.

Plates were prepared as follows. Plates were coated with 50 µl/well of a 1:100 dilution in sterile dPBS of the primary mmonoclonal anti-monkey IFN-γ antibody (UcyTech #21-43-09, Utrecht, the Netherlands), and incubated in a plastic bag at 4°C for 5-6 h. 1 h prior to use, the coating antibody was removed and the plate were blocked with cRPMI-10 in a 37°C, 5% CO₂ humidity-controlled cell culture incubator. Immediately prior to use, the blocking media was removed.

Thawing cryopreserved PBMC: Frozen vials were swirled in warm tap water (37-40°C just until barely thawed, and the 0.55 ml contents immediately transferred to 8 ml RPMI-20. Cells were washed at 350g for 13 min, and the pellet carefully resuspended in 2.0 ml cRPMI-20. A sterile 40 µl aliquot was then removed to confirm viable cell numbers, and the volume adjusted as necessary to yield a single cell suspension of 2x10⁶ cells/ml.

Pre-stimulation: equal volumes of cell suspension in cRPMI-20 and stimulants in M+ were mixed in polypropylene cell culture tubes to give the final desired concentration of all reagents. Cells were then stored in the incubator for at least 5 h, with loose caps and in a tipped position to facilitate gas exchange.

Final stimulation: After 5-6 h of incubation, the cells were spun at 400g for 10 min and the supernatants discarded. Cells were then immediately again resuspended in half cRPMI-20 and half stimulant. They were returned to the incubator for 10-20 min to allow pH stabilization. Then, cells were briefly mixed and 200 µl (200,000 cells) carefully pipetted into the appropriate wells on the blocked and emptied plates. Care was taken at all steps to ensure that the wells did not dry out. The plates were then incubated undisturbed overnight (> 16 h).

Spot development: A 1:100 dilution of secondary polyclonal anti-monkey-IFN-γ antibody (UCyTech) was made in dPBS with 2% FCS. Cells and media were flicked out of the plate; the wells were washed 8 times with dPBS-0.5% Tween 20 (Sigma), and loaded with 50 µl of the diluted secondary antibody. Plates were incubated on a rocking panel for 3 h at room temperature in a plastic bag. Plates were washed again 8 times with dPBS-0.5% Tween 20 and loaded with 50 µl/well of a 1:1000 dilution of Streptavidin-Alkaline Phosphatase conjugate (Southern Biotech #7100-04, Birmingham, AL). Plates were then incubated for an additional 2 h at room temperature in a plastic bag on the rocker panel. Finally, the plates were washed 8 times as before, followed by a single wash with distilled water and addition of 100 µl/well of chromogenic NBT-BCIP substrate (Pierce Biotech, Rockford, IL). Color was allowed to develop for 10-20 min, until the background was dark. Plates were then rinsed with at least two washes of 300 µl of distilled water, and air dryed overnight before reading.

Plate reading: Plates were read on an AID ELHR01 Elispot reader using AID ELISpot Reader v3.1.1. All wells were visually examined, and inappropriate spot counts (lint or other debris) were manually excluded. Data was saved to an Excel worksheet. Duplicate or triplicate wells were averaged, and this number multiplied by 5 to yield the final raw data in spots/million cells.

Quality control: Average viable cell recovery after freeze/thaw exceeded 95%. Runs were repeated if the media control wells averaged more than 20 spots/million, or if the ConA wells were less than 500 spots/million. Also, overall, CD4+ and CD8+ viability, as assessed using flow cytometry with 7-AAD dye exclusion and surface staining, all had to exceed 90% or the run was repeated (data not included).

**Table 1A. Experimental regimen for the prime/boost regimen in rhesus monkeys using recombinant adenoviral vectors based on serotype 5 and 35 comprising the gene encoding the CS protein of P. falciparum, and the adjuvated RTS,S as the proteinaceous antigen component.**

| **Group** | **Prime** | **Month 1** | **Month 3** | **Month 6** |
|---|---|---|---|---|
| 1 | RTS,S | RTS,S | RTS,S | |
| 2 | RTS,S | RTS,S | Ad35-CS | |
| 3 | Ad35-CS | RTS,S | RTS,S | |
| 4 | Ad35-CS | | Ad35-CS | |
| 5 | Ad35-CS | | | Ad35-CS |
| 6 | Ad5-CS | | | Ad35-CS |
| 7 | Ad35-empty | | Ad35-empty | |

**Table 1B. Blood collection schedule. CBC and cell harvests require whole blood; chemistry and ELISA's require serum. It is assumed that 1 ml of serum represents 2 ml whole blood. Only whole blood volumes are indicated. Ranges indicate where larger samples may be collected from larger monkeys. "0.5" in the CBC/Chem column indicates only CBC performed on those days. * indicates not all monkeys bled at this time point.**

| Week-Day | CBC/chem | ELISA's | Cells | TOTAL Whole blood (ml) |
|---|---|---|---|---|
| Week -4 (approx) | 1-3 | 2.5-5 | 25-35 | 28-43 |
| Week -1* | 1-3 | 2.5-5 | | 3.5-8 |
| Week0-Day0 | 1-3 | 2.5-5 | | 3.5-8 |
| 0-1 | 1-3 | 2.5 | | 3.5-5.5 |
| 0-2 | 1-3 | | | 1-3 |
| 0-3 | 1-3 | | | 1-3 |
| 1 | 1-3 | 2.5-5 | | 3.5-8 |
| 2 | 1-3 | 2.5-5 | | 3.5-8 |
| 4-0 | 1-3 | 2.5-5 | | 3.5-8 |
| 4-1 | 1-3 | 2.5 | | 3.5-5 |
| 4-2 | 1-3 | | | 1-3 |
| 4-3 | 1-3 | | | 1-3 |
| 5 | 1-3 | 2.5-5 | | 3.5-8 |
| 6 | 1-3 | 2.5-5 | | 3.5-8 |
| 8 | 0.5 | 2.5-5 | 15-35* | 18-40 |
| 10 | 0.5 | 2.5-5 | | 3-5.5 |
| 12-0 | 1-3 | 2.5-5 | | 3.5-8 |
| 12-1 | 1-3 | 2.5 | | 3.5-5.5 |
| 12-2 | 1-3 | | | 1-3 |
| 12-3 | 1-3 | | | 1-3 |
| 13 | 1-3 | 2.5-5 | | 3.5-8 |
| 14 | 1-3 | 2.5-5 | | 3.5-8 |
| 15 | 2.5 | | | 2.5 |
| 16 | 1-3 | 2.5-5 | 25-35 | 28-43 |
| 18 | 0.5 | 2.5-5 | | 3-5.5 |
| 20 | 0.5 | 2.5-5 | | 3-5.5 |
| 22 | 0.5 | 2.5-5 | | 3-5.5 |
| 25-0 | 1-3 | 2.5-5 | | 3-7.5 |
| 25-1• | 1-5 | | | 1-5 |
| 25-2• | 1-5 | | | 1-5 |
| 25-3• | 1-5 | | | 1-5 |
| 26 | 2.5-7.5 | | | 1-5 |
| 27 | 0.5 | 2.5-5 | 25-35 | 28-43 |
| 28 | 2.5 | | | 2.5 |
| 29 | 0.5 | 5 | | 5.5 |
| 31 | 0.5 | 5 | | 5.5 |
| 33 | 0.5 | 5 | | 5.5 |
| 35 | 0.5 | 5 | | 5.5 |
| 38 | 0.5 | 5 | 25-35* | 5.5-43 |
| 39 | 0.5 | 7 | | 7.5 |
| 40* | | 5-10* | 20-35* | 25-43* |
| 41* | 0.5* | 7* | | 7.5* |
| 44* | 0.5* | 2.5-5* | | 3-5.5* |
| 48* | 0.5* | 5* | | 5.5* |
| 51* | 0.5* | 5-10* | 25-35* | 28-43* |
| 52* | 0.5* | 5* | | 5.5* |

In the tables below, RTS,S is referred to simply as "RTS".

**Table 2. PfCS C-terminal-specific T cell immunity two weeks after boost: median and geometric mean IFN-γ ELISpot (in SFU/million cells) and ANOVA comparison. Different prime/boost regimens are given (left).**

| | 2 weeks after boost | |
|---|---|---|
| | Median | geo mean |
| RTS,RTS,RTS | 20 | 31 |
| RTS,RTS,Ad35 | 233 | 166 |
| Ad35,RTS,RTS | 571 | 553 |
| | | |
| Ad35,Ad35 (3 months) | 85 | 78 |
| Ad35,Ad35 (6 months) | 47 | 42 |
| Ad5,Ad35 (6 months) | 110 | 89 |
| | | |
| Ad35 empty | 2 | 2 |
| | | |
| ANOVA | | P<0.0001 |

**Table 3. Student's T-test. p-values for PfCS C-terminal-specific IFN-γ ELISpot comparison, as shown in Table 2, two weeks after boost (last vaccination).**

| | RTS,RTS, RTS | RTS,RTS, Ad35 | Ad35,RTS, RTS | | Ad35,Ad35 3 months | Ad35,Ad35 6 months |
|---|---|---|---|---|---|---|
| RTS,RTS,RTS | | | | | | |
| RTS, RTS, Ad35 | 0.05 | | | | | |
| Ad35,RTS,RTS | 0.008 | 0.03 | | | | |
| | | | | | | |
| Ad35,Ad35 3 months | 0.24 | 0.20 | 0.001 | | | |
| Ad35, Ad35 6 months | 0.70 | 0.015 | <0.0001 | | 0.19 | |
| Ad5, Ad3 | 0.16 | 0.24 | 0.0004 | | 0.80 | 0.07 |
| 6 months | | | | | | |

**Table 4. C-terminal-specific IFN-y T cell immunity three months after boost: median and geometric mean ELISpot (in SFU/million cells) and ANOVA comparison. Different prime/boost regimens are given (left).**

| | 3 months after boost | |
|---|---|---|
| | Median | geo mean |
| RTS,RTS,RTS | 8 | 9 |
| RTS,RTS,Ad35 | 35 | 49 |
| Ad35,RTS,RTS | 128 | 156 |
| | | |
| Ad35,Ad35 (3 months) | 25 | 25 |
| Ad35,Ad35 (6 months) | 15 | 15 |
| Ad5,Ad35 (6 months) | 77 | 81 |
| | | |
| Ad35 empty | 2 | 2 |
| | | |
| ANOVA | | P<0.0001 |

**Table 5. Student's T-test. p-values for ELISpot comparison, as shown in Table 4, three months after boost (last vaccination).**

| | RTS,RTS, RTS | RTS,RTS, Ad35 | Ad35,RTS, RTS | | Ad35,Ad35 3 months | Ad35, Ad35 6 months |
|---|---|---|---|---|---|---|
| RTS,RTS,RTS | | | | | | |
| RTS,RTS,Ad35 | 0.03 | | | | | |
| Ad35,RTS,RTS | 0.003 | 0.03 | | | | |
| | | | | | | |
| Ad35,Ad35 3 months | 0.15 | 0.26 | 0.0009 | | | |
| Ad35, Ad35 6 months | 0.48 | 0.07 | 0.0009 | | 0.36 | |
| Ad5, Ad35 6 months | 0.006 | 0.38 | 0.12 | | 0.04 | 0.009 |

**Table 6. B cell immunity (anti-repeat antibody titer) two weeks after final boost: median and geometric mean ELISA titer and ANOVA comparison. Different prime/boost regimens are given (left).**

| | | |
|---|---|---|
| | 2 weeks after boost | |

| | Median | geo mean |
|---|---|---|
| RTS,RTS,RTS | 3313 | 3385 |
| RTS,RTS,Ad35 | 3705 | 3400 |
| Ad35,RTS,RTS | 1737 | 2059 |
| | | |
| Ad35,Ad35 (3 months) | 295 | 336 |
| Ad35,Ad35 (6 months) | 161 | 171 |
| Ad5,Ad35 (6 months) | 339 | 347 |
| | | |
| Ad35 empty | 1 | 1 |
| | | |
| ANOVA | | P<0.0001 |

**Table 7. Student's T-test. p-values for Antibody comparison, as shown in Table 6, two weeks after final boost (last vaccination).**

| | RTS,RTS, RTS | RTS,RTS, Ad35 | Ad35,RTS, RTS | | Ad35, Ad35 3 months | Ad35, Ad35 months |
|---|---|---|---|---|---|---|
| RTS,RTS,RTS | | | | | | |
| RTS,RTS,Ad35 | 0.99 | | | | | |
| Ad35,RTS,RTS | 0.07 | 0.10 | | | | |
| | | | | | | |
| Ad35, Ad35 3 months | < 0.0001 | < 0.0001 | < 0.0001 | | | |
| Ad35,Ad35 6 months | < 0.0001 | < 0.0001 | < 0.0001 | | 0.06 | |
| Ad5,Ad35 6 months | < 0.0001 | < 0.0001 | 0.0002 | | 0.93 | 0.086 |

**Table 8. B cell immunity (antibody titer) three months after boost related to P. falciparum CS: median and geometric mean ELISA (in SFU) and ANOVA comparison. Different prime/boost regimens are given (left).**

| | 3 months after boost | |
|---|---|---|
| | Median | geo mean |
| RTS,RTS,RTS | 528 | 521 |
| RTS,RTS,Ad35 | 487 | 357 |
| Ad35,RTS,RTS | 288 | 275 |
| | | |
| Ad35,Ad35 (3 months) | 67 | 78 |
| Ad35,Ad35 (6 months) | 70 | 65 |
| Ad5,Ad35 (6 months) | 92 | 141 |
| | | |
| Ad35 empty | 0 | 1 |
| | | |
| ANOVA | | P<0.0001 |

**Table 9. Student's T-test. p-values for Antibody comparison, as shown in Table 8, three months after boost (last vaccination).**

| | RTS,RTS, RTS | RTS,RTS, Ad35 | Ad35,RTS, RTS | | Ad35,Ad35 | Ad35,Ad35 |
|---|---|---|---|---|---|---|
| RTS, RTS, RTS | | | | | | |
| RTS, RTS, Ad35 | 0.40 | | | | | |
| Ad35, RTS, RTS | 0.12 | 0.59 | | | | |
| | | | | | | |
| Ad35, Ad35 3 months | < 0.0001 | 0.005 | 0.002 | | | |
| Ad35, Ad35 6 months | < 0.0001 | 0.003 | 0.002 | | 0.32 | |
| Ad5, Ad35 6 months | 0.01 | 0.088 | | | 0.15 | 0.067 |

**Table 10. Ratio* of geometric means. T- and B cell responses. Ad35 used as a priming vaccine.**

| | **T cell response** | | **B cell response** | |
|---|---|---|---|---|
| | Ratio* (95% conf int) | Ratio* (95% conf int) | Ratio* (95% conf int) | Ratio* (95% conf int) |
| | 2 weeks | 3 months | 2 weeks | 3 months |
| Ad35,RTS,RTS | **17.7** | **17.8** | **0.61** | **0.53** |
| | (4.4-72.1) | (5.1-61.9) | (0.35-0.85) | (0.23-1.22) |
| Ad35,Ad35 | **2.5** | **2.9** | **0.10** | **0.15** |
| (3 months) | (0.5-12.9) | (0.7-12.5) | (0.05-0.18) | (0.08-0.28) |
| Ad35,Ad35 | **1.3** | **1.7** | **0.05** | **0.12** |
| (6 months) | (0.3-6.0) | (0.4-7.9) | (0.03-0.10) | (0.07-0.22) |

| | | | | |
|---|---|---|---|---|
| * RTS,RTS,RTS as reference | | | | |

**Table 11. Ratio* of geometric means. T- and B cell responses. Ad35 used as a boosting vaccine.**

| | **T cell** | **response** | **B cell** | **response** |
|---|---|---|---|---|
| | Ratio* (95% conf int) | Ratio* (95% conf int) | Ratio* (95% conf int) | Ratio* (95% conf int) |
| | 2 weeks | 3 months | 2 weeks | 3 months |
| RTS,RTS,Ad35 | **5.3** | **5.6** | **1.00** | **0.69** |
| | (1.0-29.0) | (1.2-26.1) | (0.54-1.87) | (0.27-1.77) |
| Ad5,Ad35 | **2.8** | **9.2** | **0.10** | **0.27** |
| (6 months) | (0.6-13.2) | (2.2-39.0) | (0.05-0.22) | (0.11-0.68) |

| | | | | |
|---|---|---|---|---|
| * RTS,RTS,RTS as reference | | | | |

**Table 12. PfCS N-terminal-specific IFN-y T cell immunity two weeks after final vaccination: median and geometric mean ELISpot (in SFU/million cells) and ANOVA comparison. Different prime/boost regimens are given (left).**

| | 2 weeks after boost | |
|---|---|---|
| | Median | geo mean |
| RTS,RTS,RTS | 5 | 4 |
| RTS,RTS,Ad35 | 17 | 11 |
| Ad35,RTS,RTS | 130 | 126 |
| | | |
| Ad35,Ad35 (3 months) | 68 | 78 |
| Ad35,Ad35 (6 months) | 108 | 69 |
| Ad5,Ad35 (6 months) | 68 | 72 |
| | | |
| Ad35 empty | 1 | 2 |
| | | |
| ANOVA | | P<0.0001 |

**Table 13. Student's T-test. p-values for ELISpot comparison, as shown in Table 12, two weeks after final boost (last vaccination).**

| | RTS,RTS, RTS | RTS,RTS, Ad35 | Ad35,RTS, RTS | | Ad35,Ad35 3 months | Ad35, Ad35 6 months |
|---|---|---|---|---|---|---|
| RTS, RTS, RTS | | | | | | |
| RTS, RTS, Ad35 | 0.12 | | | | | |
| Ad35,RTS,RTS | < 0.0001 | 0.002 | | | | |
| | | | | | | |
| Ad35, Ad35 3 months | 0.0002 | 0.012 | 0.39 | | | |
| Ad35, Ad35 6 months | < 0.0001 | 0.011 | 0.23 | | 0.84 | |
| Ad5, Ad35 6 months | < 0.0001 | 0.007 | 0.22 | | 0.88 | 0.94 |

**Table 14. PfCS N-terminal-specific IFN-γ T cell immunity three months after boost: median and geometric mean ELISpot (in SFU/million cells) and ANOVA comparison. Different prime/boost regimens are given (left).**

| | 3 months after boost | |
|---|---|---|
| | Median | geo mean |
| RTS,RTS,RTS | 3 | 2 |
| RTS,RTS,Ad35 | 12 | 10 |
| Ad35,RTS,RTS | 32 | 40 |
| | | |
| Ad35,Ad35 (3 months) | 25 | 32 |
| Ad35,Ad35 (6 months) | 30 | 17 |
| Ad5,Ad35 (6 months) | 63 | 55 |
| | | |
| Ad35 empty | 3 | 2 |
| | | |
| ANOVA | | P<0.0001 |

**Table 15. Student's T-test. p-values for ELISpot comparison, as shown in Table 14, three months after boost (last vaccination).**

| | RTS,RTS, RTS | RTS,RTS, Ad35 | Ad35,RTS, RTS | | Ad35,Ad35 3 months | Ad35, Ad35 6 months |
|---|---|---|---|---|---|---|
| RTS, RTS, RTS | | | | | | |
| RTS, RTS, Ad35 | 0.035 | | | | | |
| Ad35,RTS,RTS | 0.0005 | 0.066 | | | | |
| | | | | | | |
| Ad35, Ad35 3 months | 0.0005 | 0.10 | 0.73 | | | |
| Ad35, Ad35 6 months | 0.011 | 0.48 | 0.27 | | 0.39 | |
| Ad5, Ad35 6 months | < 0.0001 | 0.01 | 0.58 | | | |

**Table 16. Ratio* of geometric mean T cell response against the N-terminus of PfCS. Ad35CS used as a priming vaccine.**

| | **T cell response** | |
|---|---|---|
| | Ratio* (95% conf int) | Ratio* (95% conf int) |
| | 2 weeks | 3 months |
| Ad35,RTS,RTS | **32.4** (12.1-87.2) | **16.5** (4.7-58.3) |
| Ad35,Ad35 (3 months) | **20.0** (6.1-66.0) | **13.2** (4.1-42.3) |
| Ad35,Ad35 (6 months) | **17.9** (6.5-49.4) | **7.1** (1.7-29.3) |

| | | |
|---|---|---|
| * RTS,RTS,RTS as reference | | |

**Table 17. Ratio* of geometric mean T cell response against the N-terminus of CS. Ad35CS used as a boosting vaccine.**

| | **T cell response** | |
|---|---|---|
| | Ratio* (95% conf int) | Ratio* (95% conf int) |
| | 2 weeks | 3 months |
| RTS,RTS,Ad35 | **2.8** (0.7-10.9) | **4.1** (1.1-15.2) |
| Ad5,Ad35 (6 months) | **18.5** (7.4-46.2) | **22.4** (9.8-51.1) |

| | | |
|---|---|---|
| * RTS,RTS,RTS as reference | | |

### REFERENCES

Bruña-Romero O et al. (2001) Complete, long-lasting protection against malaria of mice primed and boosted with two distinct viral vectors expressing the same plasmodial antigen. Proc Natl Acad Sci USA 98:11491-11496
Caspers P et al. (1989) The circumsporozoite protein gene from NF54, a Plasmodium falciparum isolate used in malaria vaccine trials. Mol Biochem Parasitol 35:185-189
Clyde DF et al. (1973) Immunization of men against sporozoite-induced falciparum malaria. Am J Med Sci 266:169-177
De Jong JC et al. (1999) Adenoviruses from human immunodeficiency virus-infected individuals, including two strains that represent new candidate serotypes Ad50 and Ad51 of species B1 and D, respectively. J Clin Microbiol 37:3940-3945
Doolan DL et al. (1998) DNA vaccination as an approach to malaria control: current status and strategies. Curr Topic Microbiol Immunol 226:37-56
Estcourt MJ et al. (2002) Prime-boost immunization generates a high frequency, high-avidity CD8+ cytotoxic T lymphocyte population. Int Immunol 14:31-37
Gandon S et al. (2001) Imperfect vaccines and the evolution of pathogen virulence. Nature 414:751-756
Gordon DM et al. (1995) Safety, immunogenicity, and efficacy of a recombinantly produced Plasmodium falciparum circumsporozoite protein-hepatitis B surface antigen subunit vaccine. J Infect Dis 171:1576-1585
Hoffmann SL and Doolan DL. (2000) Malaria vaccines-targeting infected hepatocytes. Nature Med 6:1218-1219
Horn NA et al. (1995) Cancer Gene Therapy using plasmid DNA: purification of DNA for human clinical trials. Human Gene Therapy 6:565-573
Kester KE et al. (2001) RTS,S Malaria Vaccine Evaluation Group. Efficacy of recombinant circumsporozoite protein vaccine regimens against experimental Plasmodium falciparum malaria. J Infect Dis 183:640-647
Kurtis JD et al. (2001) Pre-erythrocytic immunity to Plasmodium falciparum: the case for an LSA-1 vaccine. Trends in Parasitology 17:219-223
Lalvani A et al. (1999) Potent induction of focused Thl-type cellular and humoral immune responses by RTS,S/SBAS2, a recombinant Plasmodium falciparum malaria vaccine. J Infect dis 180:1656-1664
Lockyer MJ et al. (1989) Wild isolates of Plasmodium falciparum show extensive polymorphism in T cell epitopes of the circumsporozoite protein. Mol Biochem Parasitol 37:275-280
Luke TC and Hoffman SL (2003) Rationale and plans for developing a non-replicating, metabolically active, radiation-attenuated Plasmodium falciparum sporozoite vaccine. J Exp Biol 206:3803-3808
Nardin EH et al. (2001) A totally synthetic polyoxime malaria vaccine containing Plasmodium falciparum B cell and universal T cell epitopes elicits immune responses in volunteers of diverse HLA types. J Immunol 166:481-489
Mosmann TR and Coffman RL. (1989) TH! And TH2 cells: different patterns of lymphokine secretion lead to different functional properties. Ann Rev of Immunol 7:145-173
Musti AM et al. (1983) Transcriptional mapping of two yeast genes coding for glyceraldehydes 3-phosphate dehydrogenase isolated by sequence homology with the chicken gene. Gene 25:133-143
Narum DL et al. (2001) Codon optimization of gene fragments encoding Plasmodium falciparum merzoite proteins enhances DNA vaccine protein expression and immunogenicity in mice. Infect and Immun 69:7250-7253
Nussenzweig RS et al. (1967) Protective immunity produced by the injection of X-irradiated sporozoites of Plasmodium berghei. Nature 216:160-162
Romero P et al. (1989) Cloned cytotoxic T cells recognize an epitope in the circumsporozoite protein and protect against malaria. Nature 341:323-326
Stoute JA et al. (1997) A preliminary evaluation of a recombinant circumsporozoite protein vaccine against Plasmodium falciparum malaria. N Eng J Med 336:86-91
Stoute JA et al. (1998) Long-term efficacy and immune responses following immunization with the RTS,S malaria vaccine. J Infect Dis 178:1139-1144
Sun PF et al. (2003) Protective immunity induced with malaria vaccine, RTS,S, is linked to Plasmodium falciparum circumsporozoite protein-specific CD4(+) and CD8(+) T cells producing IFN-gamma. J Immunol 171:6961-6967
Valenzuela P et al. (1979) Nucleotide sequence of the gene coding for the major protein of hepatitis B virus surface antigen. Nature 280:815-819
Vogels R et al. (2003) Replication-deficient human adenovirus type 35 vectors for gene transfer and vaccination: efficient human cell interaction and bypass of pre-existing adenovirus immunity. J Virol 77:8263
Wang R et al. (2001) Induction of CD4+ T cell-dependent CD8+ type 1 responses in humans by a malaria DNA vaccine. Proc Natl Acad Sci USA 98:10817-10822
Zevering Y et al. (1994) Effect of polymorphism of sporozoite antigens on T-cell activation. Res Immunol 145:469-476

## Claims

1. A kit of parts comprising:
- a replication-defective recombinant adenovirus in a pharmaceutically acceptable excipient, said adenovirus comprising a heterologous nucleic acid encoding a circumsporozoite (CS) antigen from a malaria-causing parasite; and
- an adjuvated proteinaceous antigen;
wherein said recombinant adenovirus is selected from the group consisting of human adenovirus serotype 11, 24, 26, 34, 35, 48, 49 and 50, and wherein said replication-defective recombinant adenovirus is a priming composition and said adjuvated proteinaceous antigen is a boosting composition.

2. A kit of parts according to claim 1, wherein said recombinant adenovirus is human adenovirus serotype 35.

3. A kit of parts according to claim 1 or 2, wherein said proteinaceous antigen comprises a CS protein, or an immunogenic fragment thereof, from a malaria-causing parasite.

4. A kit of parts according to claim 3, wherein said proteinaceous antigen comprises a hybrid protein of CS protein or an immunogenic fragment thereof fused to the surface antigen from hepatitis B virus (HBsAg), in the form of lipoprotein particles with HBsAg.

5. A kit of parts according to claim 4 wherein the proteinaceous antigen comprises RTS,S.

6. A kit of parts according to any one of claims 1-5, wherein said proteinaceous antigen is adjuvated with an adjuvant comprising QS21 and 3D-MPL.

7. A kit of parts according to claim 6, wherein the adjuvant further comprises cholesterol-containing liposomes.

8. A kit of parts according to any one of claims 1-7, wherein said malaria-causing parasite is *Plasmodium falciparum.*

9. A kit of parts according to any one of claims 1-8, wherein said heterologous nucleic acid is codon-optimized for increased production of the encoded protein in a mammal, preferably a human.

10. A kit of parts according to any one of claims 1-9, wherein said recombinant adenovirus is present in a mixture with an adjuvant.

11. A kit of parts comprising:
- a replication-defective recombinant simian, canine or bovine adenovirus in a pharmaceutically acceptable excipient, said adenovirus comprising a heterologous nucleic acid encoding a codon-optimized circumsporozolte (CS) antigen from *Plasmodium falciparum;* and
- an adjuvated proteinaceous antigen comprising RTS,S;
wherein said replication-defective recombinant Adenovirus is a priming composition and said adjuvated proteinaceous antigen is a boosting composition..

12. A kit of parts according to claim 11, wherein said proteinaceous antigen is adjuvated with an adjuvant comprising QS21 and 3D-MPL.

13. A kit of parts according to claim 12, wherein the adjuvant further comprises cholesterol-containing liposomes.

14. Use of a replication-defective recombinant adenovirus comprising a heterologous nucleic acid encoding a CS antigen from a malaria-causing parasite, and an adjuvated proteinaceous antigen in the manufacture of a medicament for the treatment or prevention of malaria, wherein said recombinant adenovirus is a simian, a canine, a bovine adenovirus or a human adenovirus serotype 11, 24, 26, 34, 35, 48, 49 or 50, and wherein said replication-defective recombinant adenovirus is used as a priming composition and said adjuvated proteinaceous antigen is used as a boosting composition.

15. Use according to claim 14, wherein said proteinaceous antigen comprises a CS protein, or an immunogenic fragment thereof, from a malaria-causing parasite.

16. Use according to claim 14 or 15, wherein said malaria-causing parasite is *Plasmodium falciparum.*

17. Use according to any one of claims 14-16, wherein said proteinaceous antigen comprises a hybrid protein of CS protein or an immunogenic fragment thereof fused to the surface antigen from hepatitis B virus (HBsAg), in the form of lipoprotein particles with HBsAg.

18. Use according to claim 17, wherein said adjuvated proteinaceous antigen comprises RTS,S.

19. Use according to any one of claims 14-18, wherein said proteinaceous antigen is adjuvated with QS21 and 3D-MPL.

20. Use according to any one of claims 14-19, wherein said heterologous nucleic acid is codon-optimized for increased production of the encoded protein in a mammal, preferably a human.

## Patentansprüche

1. Kit aus Teilen, umfassend:
- ein replikationsdefektes rekombinantes Adenovirus in einem pharmazeutisch akzeptablen Exzipienten, wobei das Adenovirus eine heterologe Nukleinsäure umfasst, die ein Circumsporozoit (CS)-Antigen aus einem Malaria-verursachenden Parasiten kodiert; und
- ein adjuvantiertes proteinöses Antigen;
wobei das rekombinante Adenovirus ausgewählt ist aus der Gruppe bestehend aus menschlichem Adenovirus Serotyp 11, 24, 26, 34, 35, 48, 49 und 50, und wobei das replikationsdefekte rekombinante Adenovirus eine Priming-Zusammensetzung ist und das adjuvantierte proteinöse Antigen eine Boost-Zusammensetzung ist.

2. Kit aus Teilen gemäß Anspruch 1, wobei das rekombinante Adenovirus menschliches Adenovirus Serotyp 35 ist.

3. Kit aus Teilen gemäß Anspruch 1 oder 2, wobei das proteinöse Antigen ein CS-Protein oder ein immunogenes Fragment davon aus einem Malaria-verursachenden Parasiten umfasst.

4. Kit aus Teilen gemäß Anspruch 3, wobei das proteinöse Antigen ein Hybridprotein des CS-Proteins oder ein immunogenes Fragment davon umfasst, das mit einem Oberflächenantigen aus Hepatitis B-Virus (HBsAg) fusioniert ist, in Form von Lipoproteinpartikeln mit HBsAg.

5. Kit aus Teilen gemäß Anspruch 4, wobei das proteinöse Antigen RTS,S umfasst.

6. Kit aus Teilen gemäß einem der Ansprüche 1 bis 5, wobei das proteinöse Antigen mit einem Adjuvans, dass QS21 und 3D-MPL umfasst, adjuvantiert wird.

7. Kit aus Teilen gemäß Anspruch 6, wobei das Adjuvans ferner Cholesterol-enthaltende Liposomen umfasst.

8. Kit aus Teilen gemäß einem der Ansprüche 1 bis 7, wobei der Malaria-verursachende Parasit Plasmodium falciparum ist.

9. Kit aus Teilen gemäß einem der Ansprüche 1 bis 8, wobei die heterologe Nukleinsäure für erhöhte Produktion des kodierten Proteins in einem Säugetier, vorzugsweise einem Menschen, Kodon-optimiert ist.

10. Kit aus Teilen gemäß einem der Ansprüche 1 bis 9, wobei das rekombinante Adenovirus in einem Gemisch mit einem Adjuvans vorliegt.

11. Kit aus Teilen, umfassend:
- ein replikationsdefektes rekombinantes Affen-, Hunde-oder Rinder-Adenovirus in einem pharmazeutisch akzeptablen Exzipienten, wobei das Adenovirus eine heterologe Nukleinsäure umfasst, die ein Kodonoptimiertes Circumsporozoit (CS)-Antigen aus Plasmodium falciparum kodiert; und
- ein adjuvantiertes proteinöses Antigen, das RTS,S umfasst;
wobei das replikationsdefekte rekombinante Adenovirus eine Priming-Zusammensetzung ist und das adjuvantierte proteinöse Antigen eine Boost-Zusammensetzung ist.

12. Kit aus Teilen gemäß Anspruch 11, wobei das proteinöse Antigen mit einem Adjuvans, das QS21 und 3D-MPL umfasst, adjuvantiert ist.

13. Kit aus Teilen gemäß Anspruch 12, wobei das Adjuvans ferner Cholesterol-enthaltende Liposomen umfasst.

14. Verwendung eines replikationsdefekten rekombinanten Adenovirus, das eine heterologe Nukleinsäure umfasst, die ein CS-Antigen aus einem Malaria-verursachenden Parasiten kodiert, und eines adjuvantierten proteinösen Antigens in der Herstellung eines Medikaments zur Behandlung oder Prävention von Malaria, wobei das rekombinante Adenovirus ein Affen-, ein Hunde-, ein Rinder-Adenovirus oder ein menschlicher Adenovirus Serotyp 11, 24, 26, 34, 35, 48, 49 oder 50 ist und wobei das replikationsdefekte rekombinante Adenovirus als Priming-Zusammensetzung verwendet wird und das adjuvantierte proteinöse Antigen als Boost-Zusammensetzung verwendet wird.

15. Verwendung gemäß Anspruch 14, wobei das proteinöse Antigen ein CS-Protein oder ein immunogenes Fragment davon aus einem Malaria-verursachenden Parasiten umfasst.

16. Verwendung gemäß Anspruch 14 oder 15, wobei der Malaria-verursachende Parasit Plasmodium falciparum ist.

17. Verwendung gemäß einem der Ansprüche 14 bis 16, wobei das proteinöse Antigen ein Hybridprotein des CS-Proteins oder ein immunogenes Fragment davon umfasst, das mit dem Oberflächenantigen aus Hepatitis B-Virus (HBsAg) fusioniert ist, in Form von Lipoproteinpartikeln mit HBsAg.

18. Verwendung gemäß Anspruch 17, wobei das adjuvantierte proteinöse Antigen RTS,S umfasst.

19. Verwendung gemäß einem der Ansprüche 14 bis 18, wobei das proteinöse Antigen mit QS21 und 3D-MPL adjuvantiert ist.

20. Verwendung gemäß einem der Ansprüche 14 bis 19, wobei die heterologe Nukleinsäure für erhöhte Produktion des kodierten Proteins in einem Säugetier, vorzugsweise einem Menschen, Kodon-optimiert ist.

## Revendications

1. Trousse d'éléments comprenant :
- un adénovirus recombinant ayant une réplication déficiente dans un excipient acceptable d'un point de vue pharmaceutique, ledit adénovirus comprenant un acide nucléique hétérologue codant un antigène circumsporozoïte (CS) provenant d'un parasite provoquant le paludisme ; et
- un antigène protéique adjuvanté ;
dans laquelle ledit adénovirus recombinant est choisi dans le groupe constitué des sérotypes 11, 24, 26, 34, 35, 48, 49 et 50 d'un adénovirus humain, et où ledit adénovirus recombinant ayant une réplication déficiente est une composition de sensibilisation et ledit antigène protéique adjuvanté est une composition de rappel.

2. Trousse d'éléments selon la revendication 1, dans laquelle ledit adénovirus recombinant est le sérotype 35 d'un adénovirus humain.

3. Trousse d'éléments selon la revendication 1 ou 2, dans laquelle ledit antigène protéique comprend une protéine CS ou un fragment immunogène de celle-ci, provenant d'un parasite provoquant le paludisme.

4. Trousse d'éléments selon la revendication 3, dans laquelle ledit antigène protéique comprend une protéine hybride de la protéine CS ou d'un fragment immunogène de celle-ci fusionnée à l'antigène de surface provenant du virus de l'hépatite B (AgHbs), sous la forme de particules lipoprotéiques avec AgHbs.

5. Trousse d'éléments selon la revendication 4, dans laquelle l'antigène protéique comprend RTS,S.

6. Trousse d'éléments selon l'une quelconque des revendications 1 à 5, dans laquelle ledit antigène protéique est adjuvanté avec un adjuvant comprenant QS21 et 3D-MPL.

7. Trousse d'éléments selon la revendication 6, dans laquelle l'adjuvant comprend en outre les liposomes contenant du cholestérol.

8. Trousse d'éléments selon l'une quelconque des revendications 1 à 7, dans laquelle ledit parasite provoquant le paludisme est *Plasmodium falciparum.*

9. Trousse d'éléments selon l'une quelconque des revendications 1 à 8, dans laquelle ledit acide nucléique hétérologue est à codons optimisés pour une production accrue de la protéine codée chez un mammifère, de préférence un humain.

10. Trousse d'éléments selon l'une quelconque des revendications 1 à 9, dans laquelle ledit adénovirus recombinant est présent dans un mélange avec un adjuvant.

11. Trousse d'éléments comprenant :
- un adénovirus simien, canin ou bovin recombinant ayant une réplication déficiente dans un excipient acceptable d'un point de vue pharmaceutique, ledit adénovirus comprenant un acide nucléique hétérologue codant un antigène circumsporozoïte (CS) à codons optimisés provenant de *Plasmodium falciparum ;* et
- un antigène protéique adjuvanté comprenant RTS, S ;
où ledit adénovirus recombinant ayant une réplication déficiente est une composition de sensibilisation et ledit antigène protéique adjuvanté est une composition de rappel.

12. Trousse d'éléments selon la revendication 11, dans laquelle ledit antigène protéique est adjuvanté avec un adjuvant comprenant QS21 et 3D-MPL.

13. Trousse d'éléments selon la revendication 12, dans laquelle l'adjuvant comprend en outre des liposomes contenant du cholestérol.

14. Utilisation d'un adénovirus recombinant ayant une réplication déficiente comprenant un acide nucléique hétérologue codant un antigène CS provenant d'un parasite provoquant le paludisme, et un antigène protéique adjuvanté dans la fabrication d'un médicament pour le traitement ou la prévention du paludisme, dans laquelle ledit adénovirus recombinant est un adénovirus simien, canin, bovin ou un adénovirus humain de sérotype 11, 24, 26, 34, 35, 48, 49 ou 50 revendication, et où ledit adénovirus recombinant ayant une réplication déficiente est utilisé en tant que composition de sensibilisation et ledit antigène protéique adjuvanté est utilisé en tant que composition de rappel.

15. Utilisation selon la revendication 14, dans laquelle ledit antigène protéique comprend une protéine CS ou un fragment immunogène de celle-ci, provenant d'un parasite provoquant le paludisme.

16. Utilisation selon la revendication 14 ou 15, dans laquelle ledit parasite provoquant le paludisme est *Plasmodium falciparum.*

17. Utilisation selon l'une quelconque des revendications 14 à 16, dans laquelle ledit antigène protéique comprend une protéine hybride d'une protéine CS ou d'un fragment immunogène de celle-ci fusionnée à l'antigène de surface du virus de l'hépatite B (AgHbs), sous la forme de particules lipoprotéiques avec AgHbs.

18. Utilisation selon la revendication 17, dans laquelle ledit antigène protéique adjuvanté comprend RTS,S.

19. Utilisation selon l'une quelconque des revendications 14 à 18, dans laquelle ledit antigène protéique est adjuvanté avec QS21 et 3D-MPL.

20. Utilisation selon l'une quelconque des revendications 14 à 19, dans laquelle ledit acide nucléique hétérologue est à codons optimisés pour une production accrue de la protéine codée chez un mammifère, de préférence un humain.
